# EUROPÄISCHE PATENTSCHRIFT

(11) **EP 1 527 073 B1**
(45) Veröffentlichungstag und Bekanntmachung des Hinweises auf die Patenterteilung: **22.08.2007**
(21) Anmeldenummer: 03766162.6
(22) Anmeldetag: 14.07.2003
(51) Int. Cl.: C07D 453/02, A61K 31/439, A61P 25/28

(54) **BENZOTHIOPHEN-, BENZOFURAN- UND INDOLHARNSTOFFE UND DEREN VERWENDUNG ALS ALPHA7-ACHR AGONISTEN**
BENZOTHIOPHENE UREA, BENZOFURANE UREA, AND INDOLE UREA, AND USE OF THE SAME AS ALPHA-7 ACHR AGONISTS
UREES DE BENZOTHIOPHENE, DE BENZOFURANNE ET D'INDOLE ET UTILISATION DE CES UREES COMME AGONISTES DES ACHR ALPHA 7

(30) Priorität: 29.07.2002 DE 10234424
(43) Veröffentlichungstag der Anmeldung: 04.05.2005
(73) Patentinhaber: Bayer HealthCare AG, 51368 Leverkusen (DE)
(72) Erfinder: FLESSNER, Timo, 42113 Wuppertal (DE); BÖSS, Frank-Gerhard, Sunninghill Berkshire SL5-7DF (GB); ERB, Christina, 65830 Kriftel (DE); HAFNER, Frank-Thorsten, 42115 Wuppertal (DE); SCHNIZLER, Katrin, 63517 Rodenbach (DE); LANG, Dieter, 42553 Velbert (DE); LUITHLE, Joachim, 42489 Wülfrath (DE); VAN KAMPEN, Marja, 40221 Düsseldorf (DE); VAN DER STAAY, Franz-Josef, 8251 BB Dronten (NL)
(86) Internationale Anmeldenummer: PCT/EP2003/007588
(87) Internationale Veröffentlichungsnummer: WO 2004/013136

(56) Entgegenhaltungen:
- WO-A-01/85727
- WO-A-02/15662
- WO-A-03/055878

## Beschreibung

Die Erfindung betrifft neue Benzothiophen-, Benzofuran- und Indolharnstoffe, Verfahren zu ihrer Herstellung sowie ihre Verwendung zur Herstellung von Arzneimitteln zur Behandlung und/oder Prophylaxe von Krankheiten und zur Verbesserung der Wahrnehmung, Konzentrationsleistung, Lernleistung und/oder Gedächtnisleistung.

Nikotinische Acetylcholin-Rezeptoren (nAChR) bilden eine große Familie von Ionenkanälen, die durch den körpereigenen Botenstoff Acetylcholin aktiviert werden (Galzi et al., Neuropharmacol. 1995, 34, 563-582). Ein funktioneller nAChR besteht aus fünf Untereinheiten, die unterschiedlich (bestimmte Kombinationen von α1-9-und β1-4,γ,δ,ε-Untereinheiten) oder identisch (α7-9) sein können. Dies führt zur Bildung einer Vielfalt von Subtypen, die eine unterschiedliche Verteilung in der Muskulatur, dem Nervensystem und anderen Organen zeigen (McGehee et al., Annu. Rev. Physiol. 1995, 57, 521-546). Aktivierung von nAChR führt zum Einstrom von Kationen in die Zelle und zur Stimulation von Nerven- oder Muskelzellen. Selektive Aktivierung einzelner nAChR-Subtypen beschränkt diese Stimulation auf die Zelltypen, die den entsprechenden Subtyp besitzen und kann so unerwünschte Nebeneffekte, wie z.B. die Stimulierung von nAChR in der Muskulatur, vermeiden. Klinische Experimente mit Nikotin und Experimente in verschiedenen Tiermodellen weisen auf eine Rolle von zentralen nikotinischen Acetylcholin-Rezeptoren bei Lern- und Gedächtnisvorgängen hin (z.B. Rezvani et al., Biol. Psychiatry 2001, 49, 258-267). Nikotinische Acetylcholinrezeptoren des alpha7-Subtyps (α7-nAChR) haben eine besonders hohe Konzentration in für Lernen und Gedächtnis wichtigen Hirnregionen, wie dem Hippocampus und dem cerebralen Cortex (Séguéla et al., J. Neurosci. 1993, 13, 596-604). Der α7-nAChR besitzt eine besonders hohe Durchlässigkeit für Calcium-Ionen, erhöht glutamaterge Neurotransmission, beeinflusst das Wachstum von Neuriten und moduliert auf diese Weise die neuronale Plastizität (Broide et al., Mol. Neurobiol. 1999, 20, 1-16).

Bestimmte *N*-(1-Aza-bicyclo[2.2.2]oct-3-yl)-heteroarylcarbonsäureamide zur Behandlung von u.a. Psychosen sind in der DE-A 37 24 059 beschrieben.

*N*-(Aza-bicycloalkyl)-heteroarylcarbonsäureamide, insbesondere *N*-(1-Aza-bicyclo-[2.2.2]oct-4-yl)-benzothiophen-3-carbonsäureamide, werden in der WO 93/15073 bzw. in der EP-A 485 962 als Zwischenstufen für die Synthese von pharmazeutisch wirksamen Verbindungen offenbart.

Aus der US 4,605,652 und der EP-A 372 335 sind beispielsweise *N*-(1-Aza-bicyclo[2.2.2]oct-3-yl)-thiophen-2-carbonsäureamid und seine gedächtnisverbessernde Wirkung bekannt.

In JP-A 14 030 084 werden 1-Azabicycloalkane zur Behandlung von u. a. Demenz, Attention Deficit Hyperactivity Disorder und Lern- und Gedächtnisstörungen beschrieben.

Die vorliegende Erfindung betrifft Verbindungen der Formel 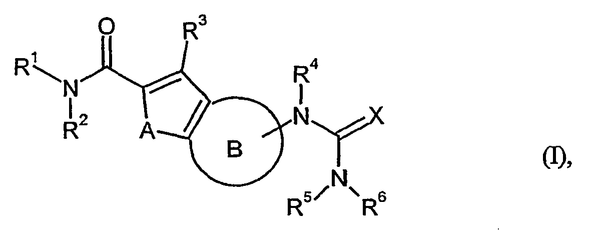 in welcher
- R¹: 1-Aza-bicyclo[2.2.2]oct-3-yl,
- R²: Wasserstoff oder C₁-C₆-Alkyl,
- R³: Wasserstoff, Halogen, Amino, Hydroxy oder C₁-C₆-Alkyl,
- R⁴: Wasserstoff, C₁-C₆-Alkyl, das gegebenenfalls durch einen Rest ausgewählt aus der Gruppe Hydroxy, Halogen, Cyano, C₁-C₆-Alkoxy, Trifluormethyl, Trifluormethoxy substituiert ist,
- R⁵: Wasserstoff oder C₁-C₆-Alkyl, oder
- R⁴ und R⁵: zusammen mit den Stickstoffatomen, an die sie gebunden sind, einen 5- bis 6-gliedrigen Heterocyclus, der gegebenenfalls mit bis zu 2 Substituenten unabhängig voneinander ausgewählt aus der Gruppe C₁-C₆-Alkyl, C₁-C₄-Acyl, Oxo, Thioxo substituiert ist,
- R⁶: (i) Wasserstoff, (ii) C₁-C₆-Alkyl, (iii) C₃-C₈-Cycloalkyl, (iv) C₆-C₁₀-Aryl, (v) 5- bis 10-gliedriges Heteroaryl, (vi) C₆-C₁₀-Arylcarbonyl wobei (ii) gegebenenfalls mit Phenyl, C₁-C₆-Alkoxycarbonyl oder C₁-C₆-Alkoxy substituiert ist und (iv), (v) und (vi) gegebenenfalls mit bis zu 3 unabhängig voneinander ausgewählten Resten aus der Gruppe C₁-C₆-Alkyl, C₁-C₆-Hydroxyalkyl, 3- bis 8-gliedriges Heterocyclyl, C₆-C₁₀-Aryl, 5- bis 10-gliedriges Heteroaryl, Hydroxy, Halogen, Cyano, C₁-C₆-Alkoxy, C₁-C₆-Acyl, Trifluormethyl, Trifluormethoxy, Nitro, Amino, C₁-C₆-Alkylamino, C₁-C₆-Acylamino substituiert sind, oder
- R⁵ und R⁶: zusammen mit dem Stickstoffatom, an das sie gebunden sind, einen 3- bis 10-gliedrigen Heterocyclus, der gegebenenfalls mit C₁-C₆-Alkyl oder C₁-C₆-Hydroxyalkyl substituiert ist,
- A: Sauerstoff, Stickstoff oder Schwefel,
- X: Sauerstoff oder Schwefel,
- der Ring B: Benzo oder Pyrido, die jeweils gegebenenfalls durch Reste aus der Reihe Halogen, Cyano, Trifluormethyl, Trifluormethoxy, Nitro, Amino, C₁-C₆-Alkyl und C₁-C₆-Alkoxy substituiert sind,
bedeuten,
sowie die Solvate, Salze oder Solvate der Salze dieser Verbindungen.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Als Salze sind im Rahmen der Erfindung physiologisch unbedenkliche Salze der erfindungsgemäßen Verbindungen bevorzugt.

Physiologisch unbedenkliche Salze der Verbindungen (I) können Säureadditionssalze der Verbindungen mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze der Verbindungen (I) sind aber auch Salze mit üblichen Basen, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabiethylamin, 1-Ephenamin oder Methylpiperidin.

Die erfindungsgemäßen Verbindungen können in stereoisomeren Formen (Enantiomere, Diastereomere) existieren. Die Erfindung betrifft daher sowohl die Enantiomeren oder Diastereomeren als auch deren jeweilige Mischungen. Diese Enantiomer- und Diastereomer-Mischungen lassen sich in bekannter Weise in die stereoisomer einheitlichen Bestandteile trennen.

Im Rahmen der vorliegenden Erfindung haben die Substituenten im Allgemeinen die folgende Bedeutung:
C₁-C₆-Alkoxy steht für einen geradkettigen oder verzweigten Alkoxyrest mit 1 bis 6, bevorzugt 1 bis 4, besonders bevorzugt 1 bis 3 Kohlenstoffatomen. Nicht-limitierende Beispiele umfassen Methoxy, Ethoxy, n-Propoxy, Isopropoxy, tert.-Butoxy, n-Pentoxy und n-Hexoxy.
C₁-C₆-Alkoxycarbonyl steht für einen geradkettigen oder verzweigten Alkoxycarbonylrest mit 1 bis 6, bevorzugt 1 bis 4 und besonders bevorzugt 1 bis 3 Kohlenstoffatomen. Nicht-limitierende Beispiele umfassen Methoxycarbonyl, Ethoxycarbonyl, n-Propoxycarbonyl, Isopropoxycarbonyl und tert.Butoxycarbonyl.
C₁-C₆-Alkyl steht für einen geradkettigen oder verzweigten Alkylrest mit 1 bis 6 bevorzugt 1 bis 4, besonders bevorzugt 1 bis 3 Kohlenstoffatomen. Nicht-limitierende Beispiele umfassen Methyl, Ethyl, n-Propyl, Isopropyl, tert.-Butyl, n-Pentyl und n-Hexyl.
C₁-C₆-Acyl steht für einen geradkettigen oder verzweigten Acylrest mit 1 bis 6, bevorzugt 1 bis 4 Kohlenstoffatomen. Nicht limitierende Beispiele umfassen Acetyl, Ethylcarbonyl, Propylcarbonyl, Isopropylcarbonyl, Butylcarbonyl, Isobutylcarbonyl, Pentylcarbonyl und Hexylcarbonyl. Bevorzugt sind Acetyl und Ethylcarbonyl.
C₃-C₈-Cycloalkyl steht für Cyclopropyl, Cyclopentyl, Cyclobutyl, Cyclohexyl, Cycloheptyl oder Cyclooctyl. Bevorzugt seien genannt: Cyclopropyl, Cyclopentyl und Cyclohexyl.
C₁-C₆-Acylamino steht für eine Amino-Gruppe mit einem geradkettigen oder verzweigten Alkanoyl-Substituenten, der 1 bis 6 Kohlenstoffatome aufweist und über die Carbonylgruppe verknüpft ist. Bevorzugt ist ein Acylamino-Rest mit 1 bis 2 Kohlenstoffatomen. Nicht-limitierende Beispiele umfassen Formamido, Acetamido, Propionamido, n-Butyramido und Pivaloylamido.
C₁-C₆-Alkylamino steht für einen geradkettigen oder verzweigten Mono- oder Dialkylaminorest mit 1 bis 6 Kohlenstoffatomen. Bevorzugt ist ein geradkettiger oder verzweigter Alkylaminorest mit 1 bis 4, besonders bevorzugt mit 1 bis 3 Kohlenstoffatomen. Nicht-limitierende Beispiele umfassen Methylamino, Ethylamino, n-Propylamino, Isopropylamino, tert.Butylamino, n-Pentylamino und n-Hexylamino, Dimethylamino, Diethylamino, Di-n-propylamino, Diisopropylamino, Di-t-butylamino, Di-n-pentylamino, Di-n-hexylamino, Ethylmethylamino, Isopropylmethylamino, n-Butylethylamino, n-Hexyl-i-pentylamino.
C₁-C₆-Hydroxyalkyl steht für einen geradkettigen oder verzweigten Hydroxyalkylrest mit 1 bis 6 Kohlenstoffatomen, der über die Alkylgruppe verknüpft ist. Bevorzugt ist ein geradkettiger oder verzweigter Hydroxyalkylrest mit 1 bis 4, besonders bevorzugt mit 1 bis 3 Kohlenstoffatomen. Nicht-limitierende Beispiele umfassen Hydroxymethyl, Hydroxyethyl, 3-Hydroxypropyl, Hydroxyisopropyl, Hydroxy-tert.butyl, 5-Hydroxypentyl und 6-Hydroxyhexyl.
3- bis 8-gliedriges Heterocyclyl steht für eine Cycloalkylgruppe mit 3 bis 8, bevorzugt 5 bis 7 Kohlenstoffatomen, wobei bis zu 2 Ringkohlenstoffatome in der Cycloalkylgruppe durch ein Stickstoffatom und/oder ein weiteres Heteroatom ausgewählt aus der Gruppe Stickstoff, Sauerstoff oder Schwefel ersetzt worden sind, und der Rest über eines der Ringstickstoffatome angebunden ist. Beispielsweise und vorzugsweise seien genannt: Pyrazolidin-1-yl, Piperazin-1-yl, Perhydro-1,4-diazepin-1-yl, Morpholin-1-yl, Thiomorpholin-1-yl, Piperidin-1-yl.
3- bis 10-gliedriges Heterocyclyl steht für eine mono- oder bicyclische Cycloalkylgruppe mit 3 bis 10, bevorzugt 5 bis 8 Kohlenstoffatomen, wobei bis zu 2 Ringkohlenstoffatome in der Cycloalkylgruppe durch ein Stickstoffatom und/oder ein weiteres Heteroatom ausgewählt aus der Gruppe Stickstoff, Sauerstoff oder Schwefel ersetzt worden sind, und der Rest über eines der Ringstickstoffatome angebunden ist. Beispielsweise und vorzugsweise seien genannt: Pyrazolidin-1-yl, Piperazin-1-yl, Perhydro-1,4-diazepin-1-yl, Morpholin-1-yl, Thiomorpholin-1-yl, Piperidin-1-yl, Aza-bicyclo[3.2.0]heptyl, Azabicyclo[3.2.1]heptyl, Azabicyclo[3.2.2]heptyl, Azabicyclo[3.2.1]octyl, Azabicyclo[3.2.2]octyl.
C₆-C₁₀-Aryl steht für einen mono- oder bicyclischen aromatischen, carbocyclischen Rest mit in der Regel 6 bis 10 Kohlenstoff-Ringatomen. Nicht-limitierende Beispiele umfassen Phenyl und Naphthyl.
C₆-C₁₀-Arylcarbonyl steht für einen mono- oder bicyclischen Arylcarbonylrest mit in der Regel 6 bis 10 Kohlenstoff-Ringatomen. Nicht-limitierende Beispiele umfassen Phenylcarbonyl und Naphthylcarbonyl.
5- bis 10-gliedriges Heteroaryl steht für einen aromatischen mono- oder bicyclischen Rest mit in der Regel 5 bis 10, vorzugsweise 5 bis 6 Ringatomen und bis zu 5, vorzugsweise bis zu 4 Hetero-Ringatomen aus der Reihe S, O und N. Nicht-limitierende Beispiele umfassen Thienyl, Furyl, Pyrrolyl, Thiazolyl, Oxazolyl, Imidazolyl, Pyridyl, Pyrimidyl, Pyridazinyl, Indolyl, Indazolyl, Benzofuranyl, Benzothiophenyl, Chinolinyl, Isochinolinyl.
Halogen steht für Fluor, Chlor, Brom und Iod. Bevorzugt sind Fluor, Chlor und Brom, besonders bevorzugt Fluor und Chlor.

Wenn Reste in den erfindungsgemäßen Verbindungen gegebenenfalls substituiert sind, können die Reste, soweit nichts anderes angegeben ist, ein- oder mehrfach gleich oder verschieden substituiert sein.

Bevorzugt sind Verbindungen der Formel (I), in welcher
- R¹: 1-Aza-bicyclo[2.2.2]oct-3-yl,
- R²: Wasserstoff oder C₁-C₄-Alkyl,
- R³: Wasserstoff, Halogen, Amino, Hydroxy oder C₁-C₄-Alkyl,
- R⁴: Wasserstoff oder C₁-C₄-Alkyl, das gegebenenfalls durch einen Rest ausgewählt aus der Gruppe Hydroxy, C₁-C₃-Alkoxy, Trifluormethyl, Trifluormethoxy substituiert ist,
- R⁵: Wasserstoff oder C₁-C₄-Alkyl, oder
- R⁴ und R⁵: zusammen mit den Stickstoffatomen, an die sie gebunden sind, einen 5- bis 6-gliedrigen Heterocyclus, der gegebenenfalls mit bis zu 2 Substituenten unabhängig voneinander ausgewählt aus der Gruppe C₁-C₆-Alkyl, C₁-C₄-Acyl, Oxo, Thioxo substituiert ist,
- R⁶: (i) Wasserstoff, (ii) C₁-C₄-Alkyl, (iii) C₅-C₆-Cycloalkyl, (iv) Phenyl, (v) 5- bis 6-gliedriges Heteroaryl, wobei (ii) gegebenenfalls mit Phenyl substituiert ist und (iv) und (v) gegebenenfalls mit bis zu 3 unabhängig voneinander ausgewählten Resten aus der Gruppe C₁-C₄-Alkyl, C₁-C₄-Hydroxyalkyl, Hydroxy, Chlor, Fluor, Cyano, C₁-C₃-Alkoxy, C₁-C₆-Acyl, Trifluormethyl, Trifluormethoxy, Amino, C₁-C₃-Alkylamino, C₁-C₃-Acylamino substituiert sind, oder
- R⁵ und R⁶: zusammen mit dem Stickstoffatom, an das sie gebunden sind, einen 5- bis 6-gliedrigen Heterocyclus, der gegebenenfalls mit C₁-C₃-Alkyl oder C₁-C₃-Hydroxyalkyl substituiert ist,
- A: Sauerstoff, Stickstoff oder Schwefel,
- X: Sauerstoff und
- der Ring B: Benzo oder Pyrido, die jeweils gegebenenfalls durch Reste aus der Reihe Chlor, Fluor, Cyano, Trifluormethyl, Trifluormethoxy, Amino, C₁-C₄ Alkyl und C₁-C₄-Alkoxy substituiert sind,
bedeuten,
sowie die Solvate, Salze oder Solvate der Salze dieser Verbindungen.

Ebenfalls bevorzugt sind Verbindungen der Formel (I), in welcher
- R¹: 1-Aza-bicyclo[2.2.2]oct-3-yl,
- R²: Wasserstoff oder C₁-C₄-Alkyl,
- R³: Wasserstoff, Halogen, Amino, Hydroxy oder C₁-C₄-Alkyl,
- R⁴: Wasserstoff, C₁-C₄-Alkyl, das gegebenenfalls durch einen Rest ausgewählt aus der Gruppe Hydroxy, Halogen, Cyano, C₁-C₃-Alkoxy, Trifluormethyl, Trifluormethoxy substituiert ist,
- R⁵: Wasserstoff oder C₁-C₄-Alkyl, oder
- R⁴ und R⁵: zusammen mit den Stickstoffatomen, an die sie gebunden sind, einen 5- bis 6-gliedrigen Heterocyclus, der gegebenenfalls mit bis zu 2 Substituenten unabhängig voneinander ausgewählt aus der Gruppe C₁-C₆-Alkyl, C₁-C₄-Acyl, Oxo, Thioxo substituiert ist,
- R⁶: (i) Wasserstoff, (ii) C₁-C₄-Alkyl, (iii) C₅-C₆-Cycloalkyl, (iv) Phenyl, (v) 5- bis 6-gliedriges Heteroaryl, (vi) C₆-C₁₀-Arylcarbonyl wobei (ii) gegebenenfalls mit Phenyl, C₁-C₄-Alkoxycarbonyl oder C₁-C₃-Alkoxy substituiert ist und (iv), (v) und (vi) gegebenenfalls mit bis zu 3 unabhängig voneinander ausgewählten Resten aus der Gruppe C₁-C₄-Alkyl, C₁-C₄-Hydroxyalkyl, 3- bis 8-gliedriges Heterocyclyl, C₆-C₁₀-Aryl, 5- bis 10-gliedriges Heteroaryl, Hydroxy, Fluor, Chlor, Cyano, C₁-C₃-Alkoxy, C₁-C₃-Acyl, Trifluormethyl, Trifluormethoxy, Nitro, Amino, C₁-C₃-Alkylamino, C₁-C₃-Acylamino substituiert sind, oder
- R⁵ und R⁶: zusammen mit dem Stickstoffatom, an das sie gebunden sind, einen 3- bis 10-gliedrigen Heterocyclus, der gegebenenfalls mit C₁-C₃-Alkyl oder C₁-C₃-Hydroxyalkyl substituiert ist,
- A: Sauerstoff oder Schwefel,
- X: Sauerstoff,
- der Ring B: Benzo oder Pyrido, die jeweils gegebenenfalls durch Reste aus der Reihe Chlor, Fluor, Cyano, Trifluormethyl, Trifluormethoxy, Amino, C₁-C₄-Alkyl und C₁-C₄-Alkoxy substituiert sind,
bedeuten,
sowie die Solvate, Salze oder Solvate der Salze dieser Verbindungen.

Ebenfalls bevorzugt sind Verbindungen der Formel (I), in welcher
- R¹: 1-Aza-bicyclo[2.2.2]oct-3-yl,
- R²: Wasserstoff,
- R³: Wasserstoff, Chlor, Fluor, Amino oder C₁-C₃-Alkyl,
- R⁴: Wasserstoff, Methyl oder Ethyl, wobei Methyl und Ethyl gegebenenfalls durch einen Rest ausgewählt aus der Gruppe Hydroxy, Methoxy, Ethoxy, Trifluormethyl, Trifluormethoxy substituiert sind, oder
- R⁴ und R⁵: zusammen mit den Stickstoffatomen, an die sie gebunden sind, einen 5- bis 6-gliedrigen Heterocyclus, der gegebenenfalls mit bis zu 2 Substituenten unabhängig voneinander ausgewählt aus der Gruppe C₁-C₃-Alkyl, C₁-C₄-Acyl, Oxo, Thioxo substituiert ist,
- R⁵: Wasserstoff oder C₁-C₃-Alkyl,
- R⁶: (i) Wasserstoff, (ii) C₁-C₄-Alkyl, (iii) Cyclopentyl, Cyclohexyl, (iv) Phenyl, (v) Benzyl, (vi) Phenethyl, wobei (iv) bis (vi) gegebenenfalls mit bis zu 3 unabhängig voneinander ausgewählten Resten aus der Gruppe Hydroxy, Chlor, Fluor, Cyano, Methoxy, Ethoxy, C₁-C₄-Acyl, Trifluormethyl, Trifluormethoxy, Amino, C₁-C₃-Alkylamino substituiert sind,
- A: Sauerstoff oder Schwefel,
- X: Sauerstoff und
- der Ring B: Benzo, das gegebenenfalls durch Reste aus der Reihe Chlor, Fluor, Cyano, Trifluormethyl, Trifluormethoxy, C₁-C₄-Alkyl, Methoxy und Ethoxy substituiert ist,
bedeuten,
sowie die Solvate, Salze oder Solvate der Salze dieser Verbindungen.

Ebenfalls bevorzugt sind Verbindungen der Formel (I), in welcher
- R¹: 1-Aza-bicyclo[2.2.2]oct-3-yl,
- R² bis R⁴: Wasserstoff,
- R⁵: Wasserstoff oder C₁-C₄-Alkyl, oder
- R⁴ und R⁵: zusammen mit den Stickstoffatomen, an die sie gebunden sind, einen 5- bis 6-gliedrigen Heterocyclus, der gegebenenfalls mit bis zu 2 Substituenten unabhängig voneinander ausgewählt aus der Gruppe C₁-C₄-Alkyl, C₁-C₄-Acyl, Oxo, Thioxo substituiert ist,
- R⁶: (i) Wasserstoff, (ii) C₁-C₄-Alkyl, (iii) C₅-C₆-Cycloalkyl, (iv) Phenyl, (v) Pyridyl, (vi) C₆-C₁₀-Arylcarbonyl wobei (ii) gegebenenfalls mit Phenyl, C₁-C₄-Alkoxycarbonyl oder C₁-C₃-Alkoxy substituiert ist und (iv), (v) und (vi) gegebenenfalls mit bis zu 3 unabhängig voneinander ausgewählten Resten aus der Gruppe C₁-C₄-Alkyl, C₁-C₄-Hydroxyalkyl, 3- bis 8-gliedriges Heterocyclyl, C₆-C₁₀-Aryl, 5- bis 10-gliedriges Heteroaryl, Hydroxy, Fluor, Chlor, Cyano, C₁-C₃-Alkoxy, C₁-C₃-Acyl, Trifluonnethyl, Trifluormethoxy, Nitro, Amino, C₁-C₃-Alkylamino, C₁-C₃-Acylamino substituiert sind, oder
- R⁵ und R⁶: zusammen mit dem Stickstoffatom, an das sie gebunden sind, einen 3- bis 10-gliedrigen Heterocyclus, der gegebenenfalls mit C₁-C₃-Alkyl oder C₁-C₃-Hydroxyalkyl substituiert ist,
- A: Sauerstoff oder Schwefel,
- X: Sauerstoff,
der Ring B Benzo,
bedeuten,
sowie die Solvate, Salze oder Solvate der Salze dieser Verbindungen.

Ebenfalls bevorzugt sind Verbindungen der Formel 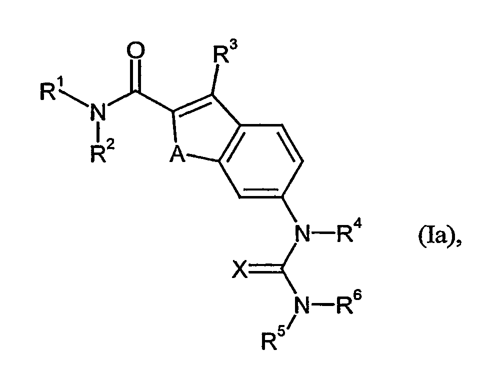 in welcher R¹ bis R⁶, A und X die oben angegebenen Bedeutungen besitzen, sowie die Solvate, Salze oder Solvate der Salze dieser Verbindungen.

Ebenfalls bevorzugt sind Verbindungen der Formel 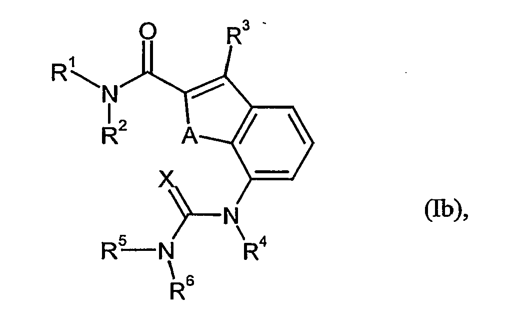 in welcher R¹ bis R⁶, A und X die oben angegebenen Bedeutungen besitzen, sowie die Solvate, Salze oder Solvate der Salze dieser Verbindungen.

Ebenfalls bevorzugt sind Verbindungen der Formel 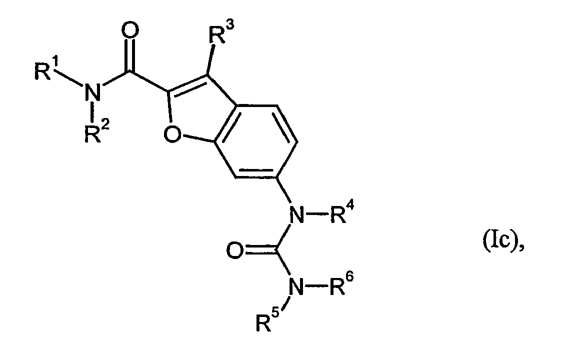 in welcher R¹ bis R⁶ die in den Ansprüchen 1 bis 6 angegebenen Bedeutungen besitzen, sowie die Solvate, Salze oder Solvate der Salze dieser Verbindungen.

Ebenfalls bevorzugt sind Verbindungen der Formel 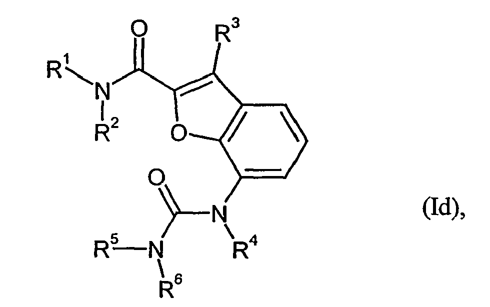 in welcher R¹ bis R⁶ die in den Ansprüchen 1 bis 6 angegebenen Bedeutungen besitzen, sowie die Solvate, Salze oder Solvate der Salze dieser Verbindungen.

Ebenfalls bevorzugt sind Verbindungen der Formeln (Ia) und (Ib) in welchen R¹ bis R⁶ die oben angegebenen Bedeutungen besitzen und A und X für Sauerstoff stehen, sowie die Solvate, Salze oder Solvate der Salze dieser Verbindungen.

Ebenfalls bevorzugt sind Verbindungen der Formel (I), in welcher R¹ (3R)-1-Aza-bicyclo[2.2.2]oct-2-yl bedeutet und R² bis R⁶, A, X und der Ring B die oben angegebenen Bedeutungen besitzen, sowie die Solvate, Salze oder Solvate der Salze dieser Verbindungen.

Ebenfalls bevorzugt sind Verbindungen der Formel (I), in welcher R² Wasserstoff bedeutet und R¹, R³ bis R⁶, A, X und der Ring B die oben angegebenen Bedeutungen besitzen, sowie die Solvate, Salze oder Solvate der Salze dieser Verbindungen.

Ebenfalls bevorzugt sind Verbindungen der Formel (I), in welcher R³ Wasserstoff, Fluor oder Methyl bedeutet und R¹, R², R⁴ bis R⁶, A, X und der Ring B die oben angegebenen Bedeutungen besitzen, sowie die Solvate, Salze oder Solvate der Salze dieser Verbindungen.

Ebenfalls bevorzugt sind Verbindungen der Formel (I), in welcher R³ Wasserstoff, Chlor oder Methyl bedeutet und R¹, R², R⁴ bis R⁶, A, X und der Ring B die oben angegebenen Bedeutungen besitzen, sowie die Solvate, Salze oder Solvate der Salze dieser Verbindungen.

Besonders bevorzugt sind Verbindungen der Formel (I), in welcher R³ Wasserstoff bedeutet und R¹, R², R⁴ bis R⁶, A, X und der Ring B die oben angegebenen Bedeutungen besitzen, sowie die Solvate, Salze oder Solvate der Salze dieser Verbindungen.

Ebenfalls bevorzugt sind Verbindungen der Formel (I), in welcher R⁴ Wasserstoff oder C₁-C₆-Alkyl, das gegebenenfalls durch einen Rest ausgewählt aus der Gruppe Hydroxy, C₁-C₆-Alkoxy, Trifluormethyl, Trifluormethoxy substituiert ist, bedeutet und R¹ bis R³, R⁵, R⁶, A, X und der Ring B die oben angegebenen Bedeutungen besitzen, sowie die Solvate, Salze oder Solvate der Salze dieser Verbindungen.

Besonders bevorzugt sind Verbindungen der Formel (I), in welcher R⁴ Wasserstoff, Methyl oder Ethyl, wobei Methyl und Ethyl gegebenenfalls durch einen Rest ausgewählt aus der Gruppe Hydroxy, Methoxy, Ethoxy, Trifluormethyl, Trifluormethoxy substituiert ist, bedeutet und R¹ bis R³, R⁵, R⁶, A, X und der Ring B die oben angegebenen Bedeutungen besitzen, sowie die Solvate, Salze oder Solvate der Salze dieser Verbindungen.

Ebenfalls bevorzugt sind Verbindungen der Formel (I), in welcher R⁵ Wasserstoff bedeutet und R¹ bis R⁴, R⁶, A, X und der Ring B die oben angegebenen Bedeutungen besitzen, sowie die Solvate, Salze oder Solvate der Salze dieser Verbindungen.

Ebenfalls bevorzugt sind Verbindungen der Formel (I), in welcher X ein Sauerstoffatom bedeutet und R¹ bis R⁶, A und der Ring B die oben angegebenen Bedeutungen besitzen, sowie die Solvate, Salze oder Solvate der Salze dieser Verbindungen.

Ebenfalls bevorzugt sind Verbindungen der Formel (I), in welcher A ein Sauerstoffatom bedeutet und R¹ bis R⁶, X und der Ring B die oben angegebenen Bedeutungen besitzen, sowie die Solvate, Salze oder Solvate der Salze dieser Verbindungen.

Ebenfalls bevorzugt sind Verbindungen der Formel (I), in welcher der Ring B Benzo bedeutet, das gegebenenfalls durch 1 bis 3 Reste aus der Reihe Halogen, Cyano, Trifluormethyl, Trifluormethoxy und C₁-C₄-Alkyl substituiert ist, und R¹ bis R⁶, A und X die oben angegebenen Bedeutungen besitzen, sowie die Solvate, Salze oder Solvate der Salze dieser Verbindungen.

Ganz besonders bevorzugt sind Kombinationen von zwei oder mehreren der oben genannten Vorzugsbereiche.

Die Erfindung betrifft weiterhin Verfahren zur Herstellung der erfindungsgemäßen Verbindungen, wonach man Verbindungen der Formel 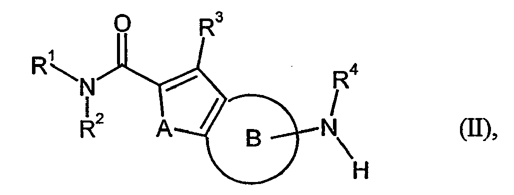 in welcher
R¹ bis R⁴, A und B die oben genannten Bedeutungen haben,
mit Verbindungen der Formel 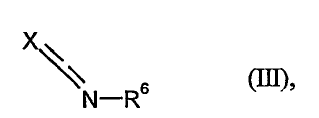 in welcher
X und R⁶ die oben genannten Bedeutungen besitzen,
und die resultierenden Verbindungen (I) gegebenenfalls mit den entsprechenden (a) Lösungsmitteln und/oder (b) Basen oder Säuren zu ihren Solvaten, Salzen oder Solvaten der Salze umsetzt.

Die Umsetzung kann in inerten Lösungsmitteln, gegebenenfalls in Gegenwart einer Base, bevorzugt in einem Temperaturbereich von 20°C bis 60°C bei Normaldruck erfolgen.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, Nitroalkane wie Nitromethan, Carbonsäureester wie Ethylacetat, Ketone wie Aceton oder 2-Butanon, gegebenenfalls N-alkylierte Carbonsäureamide wie Dimethylformamid oder Dimethylacetamid, Alkylsulfoxide wie Dimethylsulfoxid, Carbonsäurenitrile wie Acetonitril oder Heteroaromaten wie Pyridin. Bevorzugt sind Dimethylformamid oder Tetrahydrofuran.

Basen sind beispielsweise Alkalihydroxide wie Natrium- oder Kaliumhydroxid, Alkalicarbonate und -hydrogencarbonate wie Cäsiumcarbonat, Natriumhydrogencarbonat, Natrium- oder Kaliumcarbonat, oder Amide wie Lithiumdiisopropylamid, Alkylamine wie Triethylamin, Diisopropylethylamin oder DBU, bevorzugt Diisopropylethylamin oder Triethylamin.

Die Synthese von Verbindungen (I) kann nach erfolgt Satchell, Chem. Soc. Rev. 1975, 4, 231-250 oder Glebova, Russ. Chem. Rev. 1985, 54, 249-261 erfolgen.

Die Synthese von Verbindungen (II) kann nach bekannten Verfahren aus den entsprechenden Brom- bzw. Nitro-substituierten Vorläufern erfolgen. So können sie beispielsweise aus den Brom-substiuierten Aromaten durch Palladium-katalysierte Reaktionen z.B. nach J.P. Wolfe et al., Tetrahedron Lett. 1997, 38, 6367-6370 aufgebaut werden.

Die Nitrogruppe der Nitro-substituierten Vorläufer kann durch katalytische Hydrierung z.B. nach Rylander, Hydrogenation Methods, Academic Press: New York, 1967 oder Babler, Sarussi, Synth. Commun. 1981, 11, 925 reduziert werden.

Die Erfindung betrifft weiterhin Verfahren zur Herstellung der Verbindungen der Formel 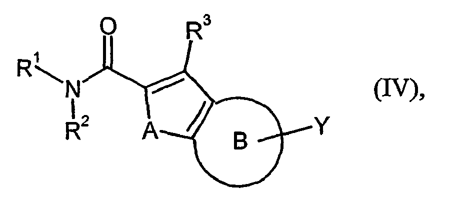 in welcher
R¹ bis R³, A und der Ring B die oben genannten Bedeutungen haben, und
- Y: für Brom, Nitro oder -NH(PG) steht, wobei PG für eine Schutzgruppe der Aminofunktion, beispielsweise tert.-Butyloxycarbonyl oder Benzyloxycarbonyl steht,
dadurch gekennzeichnet, dass man Verbindungen der Formel

R¹R²NH (V),

in welcher R¹ und R² die oben genannten Bedeutungen haben, mit einer Verbindung der Formel 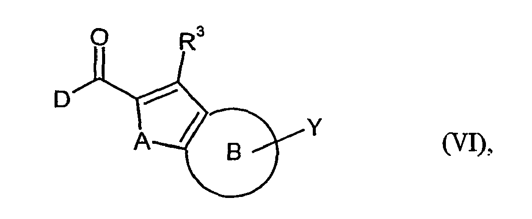 in welcher R³, A und der Ring B die oben genannten Bedeutungen haben, und
- D: für Hydroxy, Halogen, Mesyloxy oder Isobutyloxycarbonyloxy, bevorzugt Hydroxy und Chlor, steht,
umsetzt.

Die Umsetzung kann in inerten Lösungsmitteln, gegebenenfalls in Gegenwart einer Base und/oder eines Kondensationsmittels, bevorzugt in einem Temperaturbereich von 0°C bis 50°C bei Normaldruck erfolgen.

Bevorzugte inerte Lösungsmittel umfassen Dioxan, Dimethylformamid und Methylenchlorid.

Basen sind beispielsweise Alkalihydroxide wie Natrium- oder Kaliumhydroxid, Alkalicarbonate und -hydrogencarbonate wie Cäsiumcarbonat, Natriumhydrogencarbonat, Natrium- oder Kaliumcarbonat, oder Amide wie Lithiumdiisopropylamid, Alkylamine wie Triethylamin, Diisopropylethylamin oder DBU, bevorzugt Diisopropylethylamin oder Triethylamin und DBU.

Kondensationsmittel im Sinne der Erfindung sind beispielsweise Carbodiimide wie z.B. N,N'-Diethyl-, N,N,'-Dipropyl-, N,N'-DÜsopropyl-, N,N'-Dicyclohexylcarbodiimid, N-(3-Dimethylaminoisopropyl)-N'-ethylearbodiimid-Hydrochlorid (EDC), N-Cyclohexylcarbodiimid-N' -propyloxymethyl- Polystyrol (PS-Carbodiimid); Carbonylverbindungen wie Carbonyldiimidazol; 1,2-Oxazoliumverbindungen wie 2-Ethyl-5-phenyl-1,2-oxazolium-3-sulfat oder 2-tert-Butyl-5-methyl-isoxazolium-perchlorat; Acylaminoverbindungen wie 2-Ethoxy-1-ethoxycarbonyl-1,2-dihydrochinolin; weiterhin Propanphosphonsäureanhydrid, Isobutylchloroformat, Bis-(2-oxo-3-oxazolidinyl)-phosphorylchlorid, Benzotriazolyloxy-tri(dimethylamino)phosphoniumhexafluorophosphat, O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoroborat (TPTU), O-(7-Azabenzotriazol-1-yl)-N,N,N',N'-tetramethyl-uroniumhexafluorophosphat (HATU), Benzotriazol-1-yloxytris(dimethylamino)-phosphoniumhexafluoro-phosphat (BOP), und deren Mischungen.

Gegebenenfalls kann es vorteilhaft sein, das Kondensationsmittel in Gegenwart eines Hilfsnukleophils wie beispielsweise 1-Hydroxybenztriazol (HOBt) zu verwenden.

Besonders bevorzugt ist die Kombination von N-(3-Dimethylaminoisopropyl)-N-ethylcarbodiimid-Hydrochlorid (EDC), 1-Hydroxybenztriazol (HOBt) und Triethylamin in Dimethylformamid oder von O-(7-Azabenzotriazol-1-yl) *N,N,N,N-tetra*methyl-uroniumhexafluorophosphat (HATU) und Diisopropylethylamin in Dimethylformamid.

Die Verbindungen (V) und (VI) sind bekannt oder lassen sich analog bekannter Verfahren aus den entsprechenden Edukten synthetisieren (vgl. z.B. "Comprehensive Heterocyclic Chemistry", Katritzki et al., Hrsg.; Elsevier, 1996). So können beispielsweise substituierte Benzothiophen-2-carbonsäuren aus entsprechend substituierten 2-Halogenbenzaldehyden durch Reaktion mit Mercaptoessigsäuremethylester nach A. J. Bridges et al., Tetrahedron Lett. 1992, 33, 7499 und anschließender Verseifung des Esters erhalten werden: 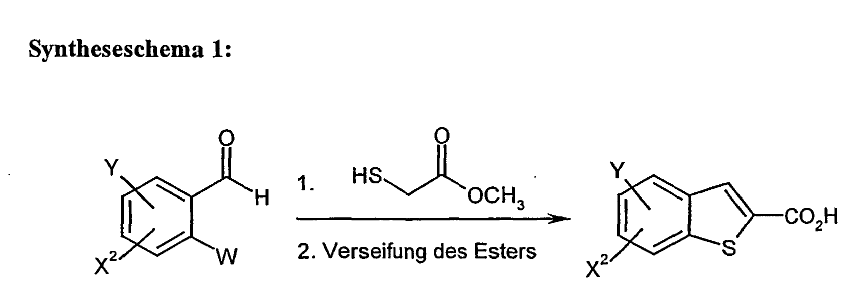 W = F, Cl, Br, NO2
X² = Halogen, Cyano, Trifluormethyl, Trifluormethoxy, Nitro, Amino, C₁-C₆-Alkyl und C₁-C₆-Alkoxy

Zur Synthese der entsprechenden Pyrido-Derivate kann man 2-Halogenbenzonitrile mit Mercaptoessigsäuremethylester zu den 3-Aminobenzothiophen-2-carbonsäureestern umsetzen:

Substituierte Benzofuran-2-carbonsäuren sind z.B. gemäß D. Bogdal et al., Tetrahedron 2000, 56, 8769 zugänglich.

Derivate von Indol-2-carbonsäuren können nach bekannten Verfahren hergestellt werden: D. A. Allen, Synth. Commun. 1999, 29, 447; C. W. Holzapfel, C. Dwyer, Heterocycles 1998, 48, 1513; M. Akazome et al., J. Org. Chem. 1994, 59, 3375.

Der Rest R³ kann beispielsweise durch die Umsetzung geeignet substituierter Ketone mit Mercaptoessigsäuremethylester entsprechend Syntheseschema 1 eingefügt werden (J. A. Valderama, C. Valderama, Synth. Commun. 1997, 27, 2143). Weitere Methoden zur Einführung von Halogen, Amino oder Hydroxy an dieser Position sind literaturbekannt: W. N. Lok, A. D. Ward, Aust. J. Chem. 1978, 31, 605; W. Reid et al., Liebigs Ann. Chem. 1980, 1424; C. M. Bonnin et al., Aust. J. Chem. 1979, 32, 883; P. Martin, T. Winkler, Helv. Chim. Acta 1994, 77, 100; A. J. Bridges, H. Zhou, J. Heterocycl. Chem. 1997, 34, 1163; J. R. Beck, J. Org. Chem. 1973, 38, 4086; T. K. Shkintova et al., Tetrahedron Lett. 2000, 41, 4973

Zur Synthese der entsprechenden Pyrido-Derivate ist ausgehend von 2-Halogenbenzonitrilen eine Reaktion mit Mercaptoessigsäuremethylester zu den 3-Aminobenzothiophen-2-carbonsäureestern möglich:

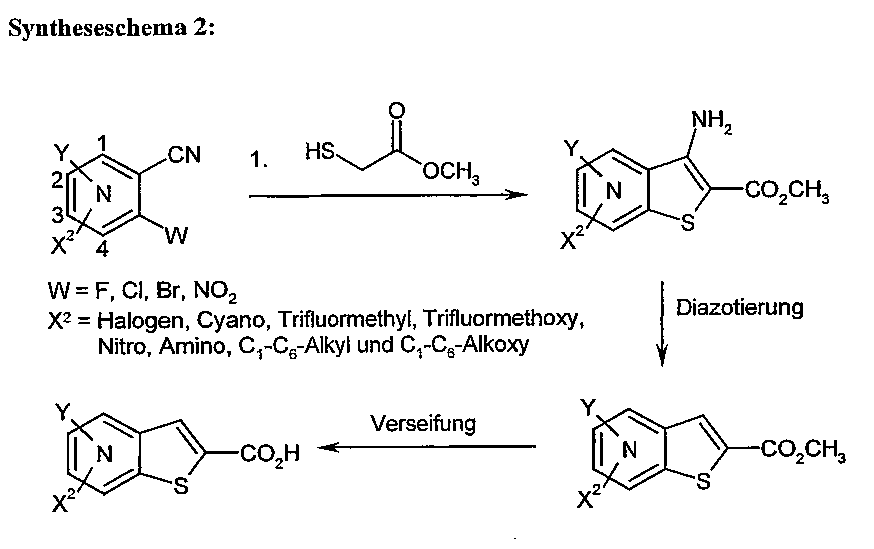

Das in den Ring gezeichnete Stickstoffatom kann an einer der Positionen 1 bis 4 im Aromaten eine CH-Gruppe ersetzen.

Die Aminofunktion kann durch Diazotierung entfernt werden. Schließlich kann der Ester zur Zielverbindung verseift werden.

Die oben beschriebenen Verfahren können durch das folgende Formelschema beispielhaft erläutert werden:

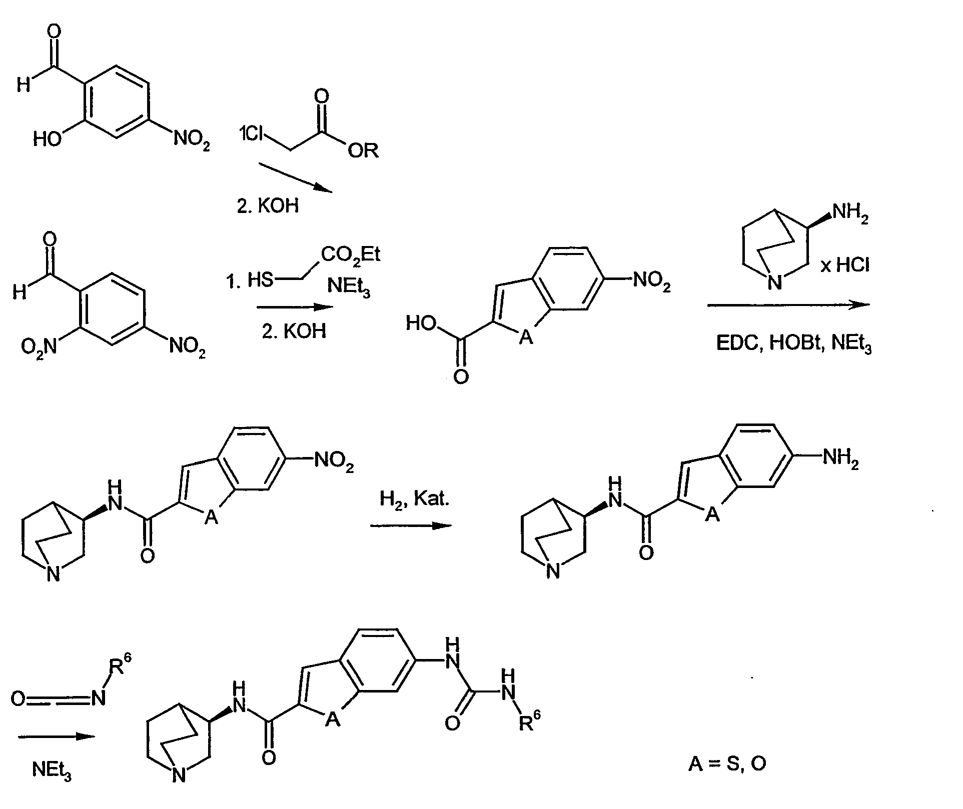

Die Abwandlung des letzten Schrittes nach folgendem Schema ermöglicht den Aufbau trisubstituierter Harnstoffe, wobei R⁵ und R⁶ mit dem Stickstoffatom, an das sie gebunden sind, einen Heterocyclus bilden können.

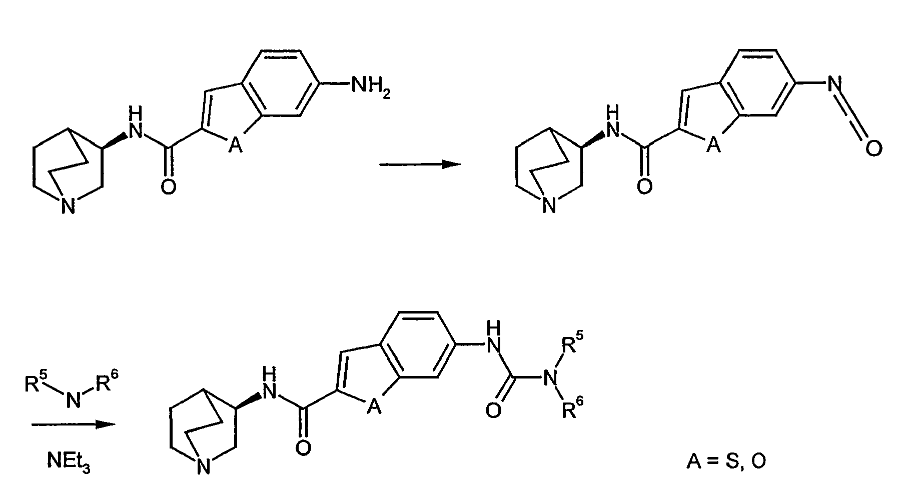

Ferner können unsymmetrisch substituierte Harnstoffe auch durch Verwendung geeigneter Phosgen-Derivate, wie z.B. p-Nitrophenylchloroformiat (Vergl.: N. Choy et al., Org. Prep. Proced. Int. 1996, 28, 173), ausgehend von unterschiedlich substituierten Aminen erhalten werden.

Die erfindungsgemäßen Verbindungen eignen sich zur Verwendung als Arzneimittel zur Behandlung und/oder Prophylaxe von Krankheiten bei Menschen und Tieren.

Sie wirken als Agonisten am α7-nAChR und zeigen ein nicht vorhersehbares, wertvolles pharmakologisches Wirkspektrum.

Die erfindungsgemäßen Verbindungen können aufgrund ihrer pharmakologischen Eigenschaften allein oder in Kombination mit anderen Wirkstoffen zur Behandlung und/oder Prävention von kognitiven Störungen, insbesondere der Alzheimerschen Krankheit eingesetzt werden. Wegen ihrer selektiven Wirkung als α7-nAChR-Agonisten eignen sie sich besonders zur Verbesserung der Wahrnehmung, Konzentrationsleistung, Lernleistung oder Gedächtnisleistung, insbesondere nach kognitiven Störungen, wie sie beispielsweise bei "Mild cognitive impairment", Alters-assoziierten Lern- und Gedächtnisstörungen, Alters-assoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Attention Deficit Hyperactivity Disorder, Alzheimerscher Krankheit, Demenz mit Lewy-Körperchen, Demenz mit Degeneration der Frontallappen einschließlich des Pick's Syndroms, Parkinsonscher Krankheit, Progressive nuclear palsy, Demenz mit corticobasaler Degeneration, Amyotropher Lateralsklerose (ALS), Huntingtonscher Krankheit, Multipler Sklerose, Thalamischer Degeneration, Creutzfeld-Jacob-Demenz, HIV-Demenz, Schizophrenie, Schizophrenie mit Demenz oder Korsakoff-Psychose auftreten.

Die erfindungsgemäßen Verbindungen können allein oder in Kombination mit anderen Wirkstoffen zur Prophylaxe und Behandlung von akuten und/oder chronischen Schmerzen (für eine Klassifizierung siehe "Classification of Chronic Pain, Descriptions of Chronic Pain Syndromes and Definitions of Pain Terms", 2. Aufl., Meskey und Begduk, Hrsg.; IASP-Press, Seattle, 1994) eingesetzt werden, insbesondere zur Behandlung von Krebs-induzierten Schmerzen und chronischen neuropathischen Schmerzen, wie zum Beispiel bei diabetischer Neuropathie, postherpetischer Neuralgie, peripheren Nervenbeschädigungen, zentralem Schmerz (beispielsweise als Folge von cerebraler Ischämie) und trigeminaler Neuralgie, und anderen chronischen Schmerzen, wie zum Beispiel Lumbago, Rückenschmerz (low back pain) oder rheumatischen Schmerzen. Daneben eignen sich diese Wirkstoffe auch zur Therapie von primär akuten Schmerzen jeglicher Genese und von daraus resultierenden sekundären Schmerzzuständen sowie zur Therapie chronischer, ehemals akuter Schmerzzustände. Die erfindungsgemäßen Verbindungen können allein oder in Kombination mit anderen Wirkstoffen zur Behandlung von Schizophrenie eingesetzt werden.

Die *in vitro*-Wirkung der erfindungsgemäßen Verbindungen kann in folgenden Assays gezeigt werden:

### 1. Bestimmung der Affinität von Testsubstanzen für α7-nAChR durch Inhibition von [³H]Methyllycaconitine-Bindung an Rattenhirnmembranen

Der [³H]-Methyllycaconitine-Bindungstest ist eine Modifikation der von Davies et al. in Neuropharmacol. 1999, 38, 679-690 beschriebenen Methode.

Rattenhirngewebe (Hippocampus oder Gesamthirn) wird in wssr. Homogenisierungspuffer (10 % w/v in Wasser, 0.32 M Sucrose, 1 mM EDTA, 0.1 mM Phenylmethylsulfonylfluorid (PMSF), 0.01 % (w/v) NaN₃, pH 7.4, 4°C) bei 600 rpm in einem Glashomogenisator homogenisiert. Das Homogenisat wird zentrifugiert (1000 x g, 4°C, 10 min) und der Überstand wird abgenommen. Das Pellet wird erneut suspendiert (20 % w/v) und die Suspension wird zentrifugiert (1000 x g, 4°C, 10 min). Die beiden Überstände werden vereinigt und zentrifugiert (15.000 x g, 4°C, 30 min). Das so erhaltene Pellet wird als P2-Fraktion bezeichnet.

Das P2-Pellet wird zweimal in Bindungspuffer (50 mM Tris-HCl, 1 mM MgCl₂, 120 mM NaCl, 5 mM KCl, 2 mM CaCl₂, pH 7.4) suspendiert und die Suspension wird zentrifugiert (15.000 x g, 4°C, 30 min).

Der Rückstand wird in Bindungspuffer zu einer Konzentration von 4 mg/ml resuspendiert und in einem Volumen von 250µl (Membranproteinmenge 0.4 mg) in Gegenwart von 2 nM [3H]-Methyl-lycaconitin, 0.1 % (w/v) BSA (bovines Serumalbumin) und verschiedenen Konzentrationen der Testsubstanz 60 min bei 21°C ink-ubiert.

Anschließend wird in Gegenwart von 1 µM α-Bungarotoxin oder 100 µM Nicotin oder 10 µM MLA (Methyllycaconitin) inkubiert. Die Inkubation wird durch Zugabe von 4 ml PBS (20 mM Na₂HPO₄, 5 mM KH₂PO₄, 150 mM NaCl, pH 7.4, 4°C) und Filtration durch Typ A/E glass fibre filters (Gelman Sciences), die vorher 3 h in 0.3 % (v/v) Polyethylenimin (PEI) eingelegt waren, beendet. Die Filter werden zweimal mit 4 ml PBS (4°C) gewaschen, und die gebundene Radioaktivität wird durch Szintillationsmessung bestimmt. Alle Tests werden als Dreifachbestimmungen durchgeführt. Aus dem IC₅₀-Wert der Verbindungen (Konzentration der Testsubstanz, bei der 50 % des am Rezeptor gebundenen Liganden verdrängt werden), der Dissoziationskonstante K_{D} und der Konzentration L von [³H]Methyllycaconitin wurde die Dissoziationskonstante Kᵢ der Testsubstanz nach der Gleichung Kᵢ = IC₅₀ / (1+L/K_{D}) bestimmt.

Anstelle von [³H]-Methyllycaconitin können auch andere α7-nAChR-selektive Radioliganden wie z.B. [¹²⁵I]-α-Bungarotoxin oder unselektive nAChR-Radioliganden gemeinsam mit Inhibitoren anderer nAChR eingesetzt werden.

Die in-vitro-Wirkdaten für die erfindungsgemäßen Verbindungen weisen einen Kᵢ-Wert von < 300 nM auf. Repräsentative in-vitro-Wirkdaten für die erfindungsgemäßen Verbindungen sind in Tabelle A wiedergegeben:

**Tabelle A**

| **Beispiel** | **Kᵢ (nM)** |
|---|---|
| 4 | 2,1 |
| 5 | 22,0 |
| 6 | 7,9 |
| 11 | 0,4 |
| 21 | 15,0 |
| 16 | 4,3 |
| 22 | < 1,00 |

Der im Folgenden beschriebene Test zeigt, dass die erfindungsgemäßen Verbindungen eine nur schwache Affinität für 5-HT₃-Rezeptoren aufweisen und somit selektiv auf α7-nAChR wirken.

### 2. Bestimmung der Affinität von Testsubstanzen für 5-HT₃-Rezeptoren durch Inhibition von [³H]GR65630-Bindung

Membranen aus HEK293 Zellen, die rekombinanten humanen 5-HT₃ Rezeptor exprimieren (RB-HS3, Receptor Biology, Inc., MD, USA), werden nach den Anweisungen des Herstellers in Inkubationspuffer (50 mM Tris-Base, pH 7.4, 5 mM MgCl₂, 0,5 mM EDTA, 0,1 % (w/v) Ascorbinsäure, 10 µM Pargyline verdünnt und in einem Volumen von 200 µl (Membranproteinmenge 3 µg) 60 min bei 21°C in Gegenwart von 0.5 nM des selektiven 5-HT₃ Rezeptor-Radioliganden [³H]-GR65630 (NET 1011, Du Pont) und unterschiedlichen Konzentrationen der Testsubstanz inkubiert. Die unspezifische Bindung wird durch Inkubation in Gegenwart von 100 µM 5-HT (5-Hydroxytryptamin) bestimmt. Die Inkubation wird beendet durch Filtration durch Typ A/E glass fibre filters (Gelman Sciences) oder GF/B Filter (Whatman), die vorher mindestens 1 h in 0.3 % (v/v) Polyethyleneimin (PEI) eingelegt waren. Die Filter werden dreimal mit 3 ml Waschpuffer (50 mM Tris-HCl, pH 7,4; 4°C) gewaschen und die gebundene Radioaktivität durch Szintillationsmessung bestimmt. Alle Tests werden in Dreifachbestimmungen durchgeführt. Aus dem IC₅₀-Wert der Verbindungen (Konzentration der Testsubstanz, bei der 50 % des am Rezeptor gebundenen Liganden verdrängt werden), der Dissoziationskonstante K_{D} und der Konzentration L von [³H]GR65630 wird die Dissoziationskonstante der Testsubstanz Kᵢ bestimmt (Kᵢ = IC₅₀ (1+L/KD)).

**Tabelle B**

| **Beispiel** | **Kᵢ (nM)** |
|---|---|
| 5 | 12000 |
| 6 | 1700 |
| 11 | 5500 |
| 21 | 1600 |
| 14 | 6900 |
| 16 | 2100 |

Die Eignung der erfindungsgemäßen Verbindungen zur Behandlung von kognitiven Störungen kann in folgenden Tiermodellen gezeigt werden:

### 3. Objekt-Wiedererkennungstest

Der Objekt-Wiedererkennungstest ist ein Gedächtnistest. Er misst die Fähigkeit von Ratten (und Mäusen), zwischen bekannten und unbekannten Objekten zu unterscheiden.

Der Test ist bei Blokland et al., NeuroReport 1998, 9, 4205-4208; A. Ennaceur et al,. Behav. Brain Res. 1988, 31, 47-59; A. Ennaceur et al., Psychopharmacology 1992, 109, 321-330; und Prickaerts et al., Eur. J. Pharmacol. 1997, 337, 125-136 beschrieben.

In einem ersten Durchgang wird eine Ratte in einer ansonsten leeren größeren Beobachtungsarena mit zwei identischen Objekten konfrontiert. Die Ratte wird beide Objekte ausgiebig untersuchen, d.h. beschnüffeln und berühren. In einem zweiten Durchgang, nach einer Wartezeit von 24 Stunden, wird die Ratte erneut in die Beobachtungsarena gesetzt. Nun ist eines der bekannten Objekte durch ein neues, unbekanntes Objekt ersetzt. Wenn eine Ratte das bekannte Objekt wiedererkennt, wird sie vor allem das unbekannte Objekt untersuchen. Nach 24 Stunden hat eine Ratte jedoch normalerweise vergessen, welches Objekt sie bereits im ersten Durchgang untersucht hat, und wird daher beide Objekte gleichstark inspizieren. Die Gabe einer Substanz mit lern- und gedächtnisverbessernder Wirkung kann dazu führen, dass eine Ratte das bereits 24 Stunden vorher im ersten Durchgang gesehene Objekt wiedererkennt. Sie wird dann das neue unbekannte Objekt ausführlicher untersuchen als das bereits bekannte. Diese Gedächtnisleistung wird in einem Diskriminationsindex ausgedrückt. Ein Diskriminationsindex von Null bedeutet, dass die Ratte beide Objekte, das alte und das neue, gleichlang untersucht; d.h. sie hat das alte Objekt nicht wiedererkannt und reagiert auf beide Objekte als wären sie neu. Ein Diskriminationsindex größer Null bedeutet, dass die Ratte das neue Objekt länger inspektiert als das alte; d.h. die Ratte hat das alte Objekt wiedererkannt.

### 4. Sozialer Wiedererkennungstest:

Der Soziale Wiedererkennungstest ist ein Test zur Prüfung der lern- oder gedächtnisverbessernden Wirkung von Testsubstanzen.

Adulte Ratten, die in Gruppen gehalten werden, werden 30 Minuten vor Testbeginn einzeln in Testkäfige gesetzt. Vier Minuten vor Testbeginn wird das Testtier in eine Beobachtungsbox gebracht. Nach dieser Adaptationszeit wird ein juveniles Tier zu dem Testtier gesetzt und 2 Minuten lang die Zeit gemessen, die das adulte Tier das juvenile Tier untersucht (Trial 1). Gemessen werden alle deutlich auf das Jungtier gerichteten Verhaltensweisen, d.h. ano-genitale Inspektion, Verfolgen sowie Fellpflege, bei denen das Alttier einen Abstand von höchstens 1 cm zu dem Jungtier hat. Danach wird das juvenile Tier herausgenommen und das adulte in seinem Testkäfig belassen (bei 24 Stunden Retention wird das Tier in seinen Heimkäfig zurückgesetzt). Vor oder nach dem ersten Test wird das adulte Testtier mit Testsubstanz behandelt. Je nach Zeitpunkt der Behandlung kann das Erlernen oder das Speichern der Information über das Jungtier durch die Substanz beeinflusst werden. Nach einem festgelegten Zeitraum (Retention) wird der Test wiederholt (Trial 2). Je größer die Differenz zwischen den in Trials 1 und 2 ermittelten Investigationszeiten, desto besser hat sich das adulte Tier an das Jungtier erinnert.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Fonnulierungen werden beispielsweise durch Verstrecken der Wirkstoffe mit Lösungsmitteln und/oder Trägerstoffen, gegebenenfalls unter Verwendung von Emulgiermitteln und/oder Dispergiermitteln hergestellt, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös. Sie kann aber auch durch Inhalation über Mund oder Nase, beispielsweise mit Hilfe eines Sprays, oder topisch über die Haut erfolgen.

Im Allgemeinen hat es sich als vorteilhaft erwiesen, Mengen von etwa 0,001 bis 10, bei oraler Anwendung vorzugsweise etwa 0,005 bis 3 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Soweit nicht anders angegeben, beziehen sich alle Mengenangaben auf Gewichtsprozente. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10 % w/v": 100 ml Lösung oder Suspension enthalten 10 g Substanz.

### Abkürzungen:

- BINAP: 2,2'-Bis-(diphenylphosphino)-1,1'-binaphthyl
- DAD: Dioden-Array-Detektor
- DMF: *N,N-*Dimethylformamid
- DMSO: Dimethylsulfoxid
- d. Th.: der Theorie
- EDTA: Ethylendiamintetraessigsäure
- eq.: Äquivalent
- ESI: Elekirospray-Ionisation (bei MS)
- HATU: *O*-(7-Azabenzotriazol-1-yl)-*N*,*N*,*N*',*N*'-tetramethyluroniumhexafluorphosphat
- HOBt: 1-Hydroxy-1H-benzotriazol x H₂O
- HPLC: Hochdruck- / Hochleistungsflüssigchromatographie
- LC-MS: Flüssigchromatographie mit gekoppelter Massenspektroskopie
- MS: Massenspektroskopie
- NMR: Kernresonanzspektroskopie
- PBS: phosphate buffered saline
- Pd₂(dba)₃: Tris-(dibenzylidenaceton)-dipalladium(0)
- RT: Raumtemperatur (20°C)
- Rₜ: Retentionszeit (bei HPLC)
- TBTU: *O*-(Benzotriazol-1-yl)-*N,N,N',N'*-tetramethyluroniumtetrafluoroborat
- THF: Tetrahydrofuran
- Tris: Tris-(hydroxymethyl)-aminomethan

### HPLC-Methode:

Instrument: HP 1100 mit DAD-Detektion; Säule: Kromasil RP-18, 60 mm x 2 mm, 3.5 µm; Eluent: A = 5 ml HClO₄/L H₂O, B = Acetonitril; Gradient: 0 min 2 % B, 0.5 min 2 % B, 4.5 min 90 % B, 6.5 min 90 % B; Fluss: 0.75 mL/min; Temp.: 30°C; Detektion: UV 210 nm.

### LC-MS-Methode:

Instrument: Micromass Platform LCZ, HP1100; Säule: Symmetry C18, 50 mm x 2.1 mm, 3.5 µm; Eluent A: Wasser + 0.05 % Ameisensäure, Eluent B: Acetonitril + 0.05 % Ameisensäure; Gradient: 0.0 min 90 % A → 4.0 min 10 % A → 6.0 min 10 % A; Ofen: 40°C; Fluss: 0.5 mL/min; UV-Detektion: 208-400 nm.

### Allgemeine Arbeitsvorschrift A:

### Synthese von 1-Benzothiophen-2-carbonsäuren

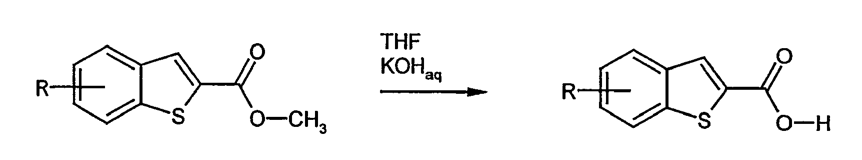

Der entsprechende 1-Benzothiophen-2-carbonsäuremethylester wird mit einer Mischung aus gleichen Teilen THF und 2 N wässriger Kaliumhydroxid-Lösung versetzt (0.28-0.47 M Lösung). Man lässt die Reaktionsmischung bei Raumtemperatur über Nacht rühren. Das THF wird unter vermindertem Druck entfernt und die wässrige Reaktionsmischung mit konzentrierter Salzsäure sauer gestellt. Der entstandene Niederschlag wird abgesaugt und im Vakuum bei 40°C getrocknet.

### Allgemeine Arbeitsvorschrift B:

### Amidknüpfung zwischen 3-Chinuklidinamin und 2-Benzothiophencarbonsäuren

1.0 eq. des entsprechenden enantiomeren 3-Chinuklidinamin-Hydrochlorids, 1.0 eq. der Carbonsäure und 1.2 eq. HATU bei 0°C in DMF werden nach Zugabe von 1.2 eq. N,N-Diisopropylethylamin 30 min. lang gerührt. Danach werden weitere 2.4 eq. N,N-Diisopropylethylamin zugegeben, und das Gemisch wird bei RT über Nacht nachgerührt.

### Ausgangsverbindungen:

### Beispiel 1A

### 7-Brom-1-benzothiophen-2-carbonsäuremethylester

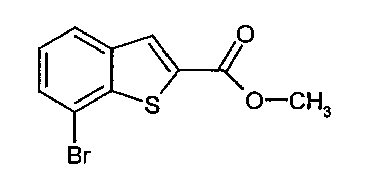

Unter Argonatmosphäre werden 8.2 g (205.7 mmol) Natriumhydrid (60 %ig in Paraffinöl) in 300 mL absolutem DMSO vorgelegt. Bei Raumtemperatur werden langsam 16.0 g (150.9 mmol) Mercaptoessigsäuremethylester zur Reaktionsmischung hinzugetropft, und man lässt bis zur Beendigung der Wasserstoffentwicklung (ca. 15 min) bei Raumtemperatur rühren. Eine Lösung von 27.8 g (137.1 mmol) 3-Brom-2-fluorbenzaldehyd in 50 mL absolutem DMSO wird bei Raumtemperatur zur Reaktionsmischung gegeben. Diese wird bis zur Beendigung der Reaktion gerührt und anschließend in Eiswasser gegossen. Der entstandene Niederschlag wird abgesaugt und über Nacht im Vakuum bei 40°C getrocknet. Man erhält 20.57 g eines Gemisches aus der Titelverbindung und der korrespondierenden Säure (ca. 1:1).

### Beispiel 2A

### 6-Brom-1-benzothiophen-2-carbonsäuremethylester

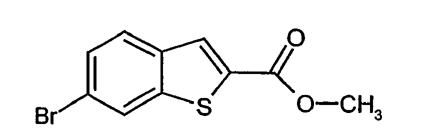

Unter Argonatmosphäre werden 1.93 g (48.3 mmol) Natriumhydrid (60 %ig in Paraffinöl) in 60 mL absolutem DMSO vorgelegt. Bei Raumtemperatur werden langsam 3.76 g (35.5 mmol) Mercaptoessigsäuremethylester zur Reaktionsmischung hinzugetropft und man lässt bis zur Beendigung der Wasserstoffentwicklung (ca. 15 min) bei Raumtemperatur rühren. Eine Lösung von 6.54 g (32.2 mmol) 4-Brom-2-fluorbenzaldehyd und 15 mL absolutem DMSO werden bei Raumtemperatur zur Reaktionsmischung gegeben. Diese wird bis zur Beendigung der Reaktion gerührt und anschließend in Eiswasser gegossen. Der entstandene Niederschlag wird abgesaugt und über Nacht im Vakuum bei 40°C getrocknet. Man erhält 4.06 g (46.4 % d.Th) der Titelverbindung.
¹H-NMR (200.1 MHz, DMSO-d₆): δ = 8.42 (d, 1H), 8.22 (s, 1H), 7.98 (d, 1H), 7.65 (dd, 1H), 3.90 (s, 3H)
HPLC: Rₜ = 5.3 min
MS (ESIpos): *m*/*z* = 270 (M⁺), 288 (M+NH₄)⁺, 305 (M+N₂H₇)⁺

### Beispiel 3A

### 6-Nitro-1-benzothiophen-2-carbonsäuremethylester

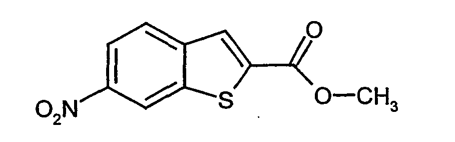

In 6 mL DMSO werden sukzessive 2 g (10.2 mmol) 2,4-Dinitrobenzaldehyd, 1.08 g (10.2 mmol) Mercaptoessigsäuremethylester und 2.84 mL (20.4 mmol) Triethylamin gelöst. Die Reaktionslösung wird 1 h auf 80°C erhitzt und dann auf 200 mL Eiswasser gegeben. Mit Dichlormethan wird mehrmals extrahiert. Die vereinigten organischen Phasen werden über Natriumsulfat getrocknet und im Vakuum eingeengt. Chromatographie (Laufmittel: Dichlormethan) an Kieselgel liefert 1.12 g (46.1 % d. Th.) der Titelverbindung.
¹H-NMR (300 MHz, CDCl₃): δ = 8.80 (d, 1H), 8.27 (dd, 1H), 8.13 (s, 1H), 8.00 (d, 1H), 3.99 (s, 3H)
HPLC: Rₜ = 4.7 min
MS (ESIpos): *m*/*z* = 255 (M+NH₄)⁺

### Beispiel 4A

### 6-Brom-1-benzothiophen-2-carbonsäure

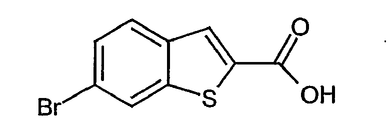

Aus 4.0 g (14.8 mmol) 6-Brom-1-benzothiophen-2-carbonsäuremethylester werden entsprechend der allgemeinen Arbeitsvorschrift A 3.55 g (93.5 % d. Th.) des gewünschten Produkts erhalten.
¹H-NMR (400.1 MHz, DMSO-d₆): δ = 13.48 (s, 1H, br), 8.38 (s, 1H), 8.22 (s, 1H), 7.96 (d, 1H), 7.63 (m, 1H)
HPLC: Rₜ = 4.5 min.

### Beispiel 5A

### 7-Brom-1-benzothiophen-2-carbonsäure

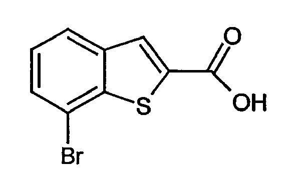

Aus 10.0 g (36.9 mmol) 7-Brom-1-benzothiophen-2-carbonsäuremethylester werden entsprechend der allgemeinen Arbeitsvorschrift A 8.99 g (91.0 % d. Th.) des gewünschten Produkts erhalten.
¹H-NMR (200.1 MHz, DMSO-d₆): δ = 13.76 (s, 1H, br), 8.28 (s, 1H), 8.07 (d, 1H), 7.78 (d, 1H), 7.46 (dd, 1H)
HPLC: 99.1 %, Rₜ = 4.4 min.

### Beispiel 6A

### 6-Nitro-1-benzothiophen-2-carbonsäure

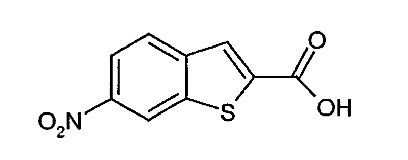

Aus 1.4 g (5.90 mmol) 6-Nitro-1-benzothiophen-2-carbonsäuremethylester werden entsprechend der allgemeinen Arbeitsvorschrift A 1.20 g (91.4 % d. Th.) des gewünschten Produkts erhalten.
¹H-NMR (200.1 MHz, DMSO-d₆): δ = 8.91 (d, 1H), 8.14 (dd, 1H), 8.02 (d, 1H), 7.69 (s, 1H)
HPLC: Rₜ = 4.1 min
MS (ESIpos): *m*/*z* = 241 (M+NH₄)⁺

### Beispiel 7A

### 6-[(tert.-Butoxycarbonyl)-amino]-1-benzothiophen-2-carbonsäure

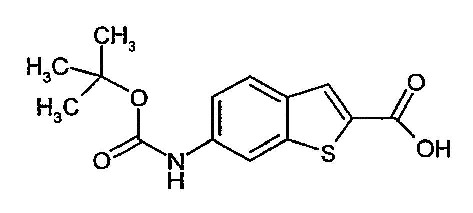

400 mg (2.07 mmol) 6-Amino-1-benzothiophen-2-carbonsäure und 1.36 g (6.21 mmol) Pyrokohlensäure-di-tert.-butylester werden in einer Mischung aus 5 mL Dioxan und 10 mL wässriger 1 N Natriumhydrogencarbonat-Lösung 18 h bei Raumtemperatur gerührt. Die Lösung wird mit 10 %iger wässriger Zitronensäure-Lösung auf pH 5-6 eingestellt und viermal mit Essigsäureethylester extrahiert. Die vereinigten organischen Phasen werden über Natriumsulfat getrocknet und eingeengt. Der Rückstand wird an Kieselgel chromatographiert (Laufmittel: Dichlormethan/Methanol). Man erhält 214 mg (30 % d. Th.) der Titelverbindung.
MS (ESIpos): *m*/*z* = 316 (M+Na)⁺

### Beispiel 8A

### N-[(3R)-1-Azabicyclo[2.2.2]oct-3-yl]-7-brom-1-benzothiophen-2-carboxamid-Hydrochlorid

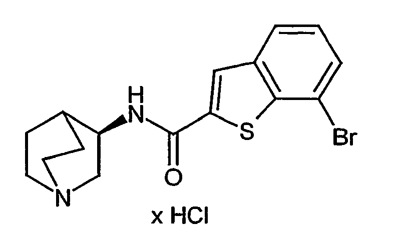

903.8 mg (3.52 mmol) 7-Brom-l-benzothiophen-2-carbonsäure, 700.0 mg (3.52 mmol) *R*-Aminochinulidin-Dihydrochlorid, 1604.0 mg (4.22 mmol) HATU, 1635.7 mg (12.66 mmol) N,N-Diisopropylethylamin und 7.0 mL DMF werden gemäß der allgemeinen Arbeitsvorschrift B umgesetzt. Das Reaktionsgemisch wird durch präparative HPLC gereinigt. Das Produkt wird in einem 1:1-Gemisch aus 4 M HCl in Dioxan und 1 N Salzsäure gelöst, anschließend eingeengt und im Hochvakuum getrocknet. Es werden 1087 mg (77 % d. Th.) der Titelverbindung erhalten.
¹H-NMR (200.1 MHz, DMSO-d₆): δ = 10.01 (s, 1H, br), 9.15 (d, 1H), 8.47 (s, 1H), 8.02 (m, 1H), 7.74 (m, 1H), 7.43 (dd, 1H), 4.34 (m, 1H), 3.80-3.10 (m, 6H), 2.22 (m, 1H), 2.14 (m, 1H), 1.93 (m, 2H), 1.78 (m, 1H)
HPLC: Rₜ = 4.1 min
MS (ESIpos): *m*/*z* = 367 (M+H, ⁸¹Br)⁺, 365 (M+H, ⁷⁹Br)⁺

### Beispiel 9A

### N-[(3R)-1-Azabicyclo[2.2.2]oct-3-yl]-6-brom-1-benzothiophen-2-carboxamid-Hydrochlorid

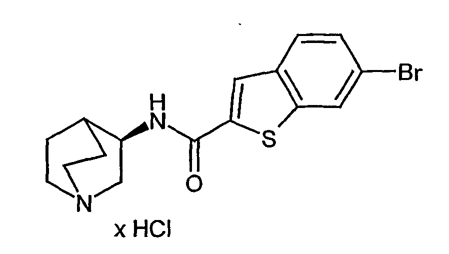

900.0 mg (3.50 mmol) 4-Brom-1-benzothiophen-2-carbonsäure, 697.0 mg (3.50 mmol) *R*-Aminochinuklidin-Dihydrochlorid, 1597.1 mg (4.20 mmol) HATU, 1628.7 mg (12.60 mmol) N,N-Diisopropylethylamin und 8.0 mL DMF werden gemäß der allgemeinen Arbeitsvorschrift B umgesetzt. Das Reaktionsgemisch wird durch präparative HPLC gereinigt. Das Produkt wird in einem 1:1-Gemisch aus 4 M HCl in Dioxan und 1 N Salzsäure gelöst und anschließend eingeengt. Die Umkristallisation aus Methanol/Ethanol (1:10) ergibt 594 mg (42.1 % d. Th.) der Titelverbindung.
¹H-NMR (300.1 MHz, DMSO-d₆): δ = 9.81 (s, 1H, br), 8.76 (m, 1H), 8.33 (s, 1H), 8.22 (s, 1H), 7.91 (d, 1H), 7.59 (dd, 1H), 4.15 (m, 1H), 3.51-2.93 (m, 6H), 2.12-1.92 (m, 2H), 1.79 (m, 2H), 1.58 (m, 1H)
HPLC: Rₜ = 4.1 min
MS (ESIpos): *m*/*z* = 366 (M, ⁸¹Br)⁺, 364 (M, ⁷⁹Br)⁺

### Beispiel 10A

### N-[(3R)-1-Azabicyclo[2.2.2]oct-3-yl]-6-nitro-1-benzothiophen-2-carboxamid-Hydrochlorid

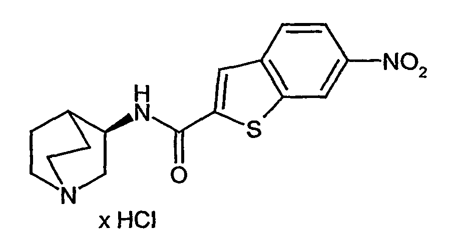

290.0 mg (1.30 mmol) 6-Nitro-1-benzothiophen-2-carbonsäure, 258.7mg (1.30 mmol) *R*-Aminochinuklidin-Dihydrochlorid, 592.8 mg (1.56 mmol) HATU, 604.5 mg (4.68 mmol) N,N-Diisopropylethylamin und 2.0 mL DMF werden gemäß der allgemeinen Arbeitsvorschrift B umgesetzt. Das Reaktionsgemisch wird durch präparative HPLC gereinigt, mit 1 N Salzsäure versetzt und anschließend eingeengt. Die Umkristallisation aus Isopropanol ergibt 297 mg (62.1 % d. Th.) der Titelverbindung.
¹H-NMR (400 MHz, MeOD-d₄): δ = 8.95 (s, 1H), 8.28 (dd, 1H), 8.20 (s, 1H), 811 (d, 1H), 4.46 (m, 1H), 3.87 (m, 1H), 3.52-3.22 (m, 5H), 2.40 (m, 1H), 2.28 (m, 1H), 2.11 1 (m, 2H), 1.98 (m, 1H)
HPLC: Rₜ = 3.8 min.
MS (ESIpos): *m*/*z* = 332 (M+H)⁺.

### Beispiel 11A

### N-[(3R)-1-Azabicyclo[2.2.2]oct-3-yl]-7-amino-1-benzothiophen-2-carboxamid-Dihydrochlorid

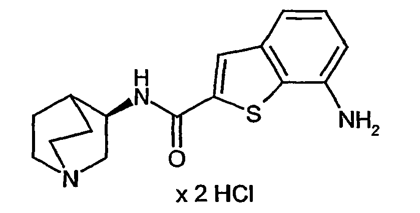

87 mg (0.22 mmol) N-[(3R)-1-Azabieyclo[2.2.2]oct-3-yl]-3-brom-1-benzothiophen-2-carboxamid-Hydrochlorid, 47.1 mg (0.26 mmol) Benzophenonimin, 12.1 mg (0.02 mmol) *rac*-BINAP, 45.8 mg (0.48 mmol) Natrium-tert.-butylat und 6.0 mg (0.01 mmol) Pd₂(dba)₃ werden unter Argon in einen ausgeheizten Kolben gegeben. 1.5 mL Toluol werden hinzugefügt, und die Reaktionsmischung wird auf 80°C erhitzt. Nach 30 min werden 0.5 mL THF und nach 6 h weitere 6.0 mg (0.01 mmol) Pd₂(dba)₃ hinzugefügt. Nach weiteren 6 h erfolgt eine Filtration (0.45 µm-Filter) und anschließend eine Reinigung mittels präparativer HPLC. Das entstandene Benzophenonimin-Addukt wird in einem 1:1-Gemisch aus THF und Methanol unter Zugabe von 20 Vol.-% 1 N Salzsäure gelöst. Nach 1 h bei Raumtemperatur wird das Reaktionsgemisch eingeengt. Der entstandene Feststoff wird mit Acetonitril verrührt und filtriert. Nach Trocknung im Hochvakuum erhält man 17 mg (21 % d. Th.) der Titelverbindung.
¹H-NMR (400.1 MHz, D₂O): δ = 8.11 (s, 1H), 7.89 (d, 1H), 7.53 (dd, 1H), 7.37 (d, 1H), 4.48 (m, 1H), 3.87 (m, 1H), 3.52-3.30 (m, 5H), 2.44 (m, 1H), 2.27 (m, 1H), 2.12 (m, 2H), 2.00 (m, 1H)
HPLC: Rₜ = 2.9 min
MS (ESIpos): *m*/*z* = 302 (M+H)⁺

### Beispiel 12A

### 6-Amino-N-[(3R)-1-azabicyclo[2.2.2]oct-3-yl]-1-benzothiophen-2-carboxamid-Dihydrochlorid

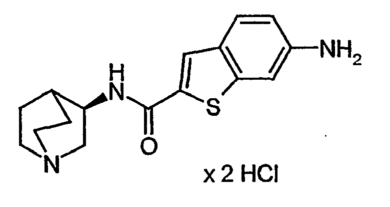

### Methode A):

15 mg (0.05 mmol) 6-[(tert.-Butoxycarbonyl)-amino]-1-benzothiophen-2-carbonsäure, 10.2 mg (0.05 mmol) *R*-Aminochinuklidin-Dihydrochlorid, 21.4 mg (0.06 mmol) HATU, 21.8 mg (0.17 mmol) N,N-Diisopropylethylamin und 1 mL DMF werden gemäß der allgemeinen Arbeitsvorschrift B umgesetzt. Das Reaktionsgemisch wird durch präparative HPLC gereinigt. Das Produkt wird mit 5 mL 4 M HC1 in Dioxan versetzt und 30 min bei Raumtemperatur gerührt. Es wird eingeengt und im Hochvakuum getrocknet. Man erhält 17 mg (98 % d. Th.) der Titelverbindung.

### Methode B):

247 mg (0.67 mmol) N-[(3R)-1-Azabicyclo[2.2.2]oct-3-yl]-6-nitro-1-benzothiophen-2-carboxamid-Hydrochlorid werden in 1.6 mL 1 N Salzsäure und 4.3 mL Methanol suspendiert und unter Argon mit 25.6 mg Palladium auf Kohle (5 %ig) versetzt. Unter einer Wasserstoffatmosphäre (Normaldruck) wird für 2 h gerührt. Der Kolbeninhalt wird über Kieselgur filtriert und im Vakuum zur Trockene eingeengt. Man erhält 241 mg (95.6 % d. Th.) der Titelverbindung.

### Methode C):

Zu einer Mischung aus 730 mg (1.76 mmol) N-[(3R)-1-Azabicyclo[2.2.2]oct-3-yl]-6-brom-l-benzothiophen-2-carboxamid-Hydrochlorid, 638.9 mg (3.53 mmol) Benzophenonimin, 109.8 mg (0.18 mmol) *rac*-BINAP, 508.2 mg (5.29 mmol) Natrium-tert.-butylat und 161.4 mg (0.18 mmol) Pd₂(dba)₃ werden 10 mL einer 1:1-Mischung aus THF und Toluol hinzugefügt, und die Reaktionsmischung wird über Nacht auf 85°C erhitzt. Der Kolbeninhalt wird auf ca. 7 mL eingeengt und mittels präparativer HPLC aufgereinigt. Das entstandene Benzophenonimin-Addukt wird in einer Mischung aus 5 mL Methanol und 3 mL 1 N Salzsäure gelöst und 1 h bei Raumtemperatur gerührt. Nach dem Einengen der Lösung erfolgen eine Umkristallisation aus Methanol/Diethylether sowie eine weitere Reinigung mittels präparativer HPLC. Man versetzt die Produktfraktionen mit 1 N Salzsäure. Nach Einengen und Trocknen im Hochvakuum erhält man 67 mg (10.1 % d. Th.) der Titelverbindung.
¹H-NMR (400.1 MHz, D₂O): δ = 7.95 (m, 2H), 7.88 (m, 1H), 7.32 (m, 1H), 4.37 (m, 1H), 3.80-3.69 (m, 2H), 3.40-3.18 (m, 4H), 2.32 (m, 1H), 2.16 (m, 1H), 2.00 (m, 2H), 1.89 (m, 1H)
HPLC: Rₜ = 2.7 min
MS (ESIpos): *m*/*z =* 302 (M+H)⁺

### Beispiel 13A

### 2-(Hydroxymethyl)-5-nitrophenol

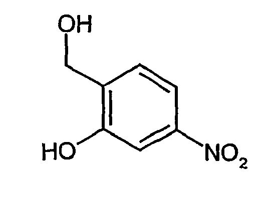

10.0 g (54.6 mmol) 4-Nitrosalicylsäure werden in 100 mL THF vorgelegt. Unter Eiskühlung versetzt man mit 109 mL 1 M Boran-THF-Komplex und lässt über Nacht bei Raumtemperatur nachrühren. Es wird eingeengt und der Niederschlag abgesaugt. Der Feststoff wird in Essigsäureethylester gelöst und über Magnesiumsulfat getrocknet. Nach dem Einengen und dem Trocknen im Hochvakuum wird die Titelverbindung direkt weiter umgesetzt.
LC-MS (ESIpos): *m*/*z* = 169 (M⁺)

### Beispiel 14A

### 2-Hydroxy-4-nitrobenzaldehyd

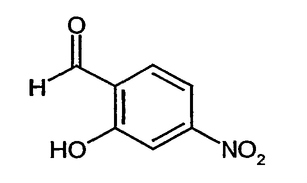

6.0 g (35.5 mmol) 2-(Hydroxymethyl)-5-nitrophenol und 3.1 g (35.5 mmol) aktiviertes Mangan(IV)oxid werden in 100 mL Chloroform für 20 h am Rückfluss erhitzt. Es wird über Kieselgur filtriert, eingeengt und im Hochvakuum getrocknet. Die Titelverbindung wird direkt weiter umgesetzt.
MS (ESIpos): *m*/*z* =167 (M⁺)

### Beispiel 15A

### 6-Nitro-1-benzofuran-2-carbonsäure

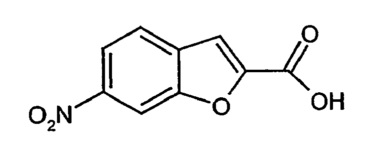

5.8 g (34.7 mmol) 2-Hydroxy-4-nitrobenzaldehyd, 1.28 g (3.5 mmol) Tetrabutylammoniumiodid und 19.2 g (138.8 mmol) Kaliumcarbonat werden vermischt, mit 7.9g (72.9 mmol) Chloressigsäuremethylester versetzt und für 12 h auf 130°C erhitzt. Es werden 100 mL THF hinzugefügt und unter Eiskühlung 11.7 g (208.2 mmol) Kaliumhydroxid zugesetzt. Nach der Zugabe von 100 mL Wasser wird 20 h bei Raumtemperatur gerührt. Der pH-Wert wird mittels konz. Salzsäure auf pH 1 eingestellt. Es wird mit Essigsäureethylester extrahiert. Die organische Phase wird mit Wasser gewaschen und über Natriumsulfat getrocknet. Nach Zugabe von Kieselgel wird eingeengt und an Kieselgel chromatographiert (Laufinittel: Toluol/Methanol/Essigsäure). Nach dem Einengen der Produktfraktionen und dem Trocknen im Vakuum erhält man 1.31 g (18.2 % d. Th.) der Titelverbindung.
HPLC: Rₜ = 3.8 min
MS (ESIpos): *m*/*z* = 225 (M+NH₄)⁺

### Beispiel 16A

### N-[(3R)-1-Azabicyclo [2.2.2] oct-3-yl]-6-nitro-1-benzofuran-2-carboxamid

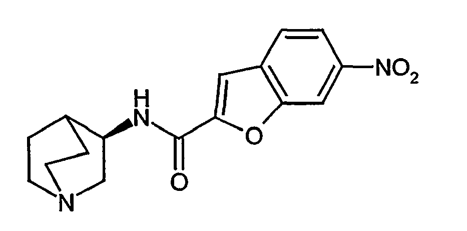

1.3 g (5.02 mmol) 6-Nitro-1-benzofuran-2-carbonsäure, 1.0 g (5.02 mmol) *R-*Aminochinuklidin-Dihydrochlorid, 2.29 g (6.02 mmol) HATU, 2.34 g (18.07 mmol) N,N-Diisopropylethylamin und 10 mL DMF werden gemäß der allgemeinen Arbeitsvorschrift B umgesetzt. Es wird im Vakuum eingeengt, mit 1 N Natronlauge und mit Essigsäureethylester extrahiert, die organische Phase über Natriumsulfat getrocknet, eingeengt, und der Rückstand in Methanol gelöst. Man versetzt mit Dowex 50WX2-200 Ionenaustauscher-Harz und engt am Rotationsverdampfer innerhalb 1 h ein. Das Harz wird sukzessive mit Methanol, DMF, Methanol, THF, Methanol, Dichlormethan, Methanol und 10 % Triethylamin in Methanol gewaschen. Die Produktfraktion wird eingeengt. Man erhält 1.75 g (99 % d. Th.) der Titelverbindung.
HPLC: Rₜ = 3.6 min.
MS (ESIpos): *m*/*z* = 316 (M+H)⁺

### Beispiel 17A

### 6-Amino-N-[(3R)-1-azabicyclo[2.2.2]oct-3-yl]-1-benzofuran-2-carboxamid

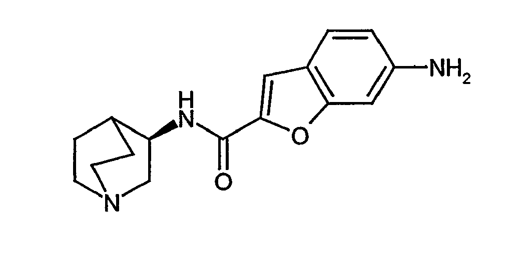

1.55 g (4.92 mmol) N-[(3R)-1-Azabicyclo[2.2.2]oct-3-yl]-6-nitro-1-benzofuran-2-carboxamid werden in 15 mL einer 2 M Zinn(II)chlorid-Lösung in DMF gelöst und über Nacht bei Raumtemperatur gerührt. Man versetzt mit Dowex 50WX2-200 Ionenaustauscher-Harz und engt am Rotationsverdampfer innerhalb 1 h ein. Das Harz wird sukzessive mit Wasser, DMF, Methanol, Dichlormethan, Methanol und 10 % Triethylamin in Methanol gewaschen. Die Produktfraktion wird eingeengt und an Kieselgel chromatographiert (Laufmittel: Dichlormethan/Methanol/Triethylamin). Nach dem Einengen im Vakuum erhält man 642.8 mg (45.8 % d. Th.) der Titelverbindung.
HPLC: Rₜ = 2.6 min
MS (ESIpos): *m*/*z* = 286 (M+H)⁺.

### Ausführungsbeispiele:

### Beispiel 1

### 6-[(Anilinocarbonyl)amino]-N-[(3R)-1-azabicyclo[2.2.2]oct-3-yl]-1-benzothiophen-2-carboxamid-Hydrochlorid

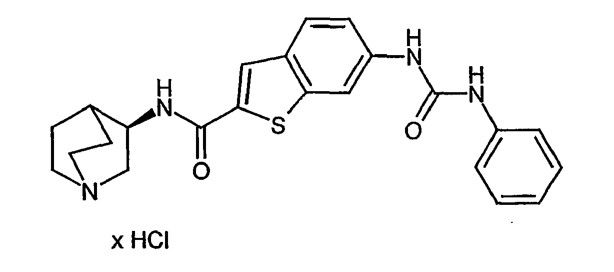

Eine Lösung von 80 mg (0.21 mmol) 6-Amino-N-[(3R)-1-azabicyclo[2.2.2]oct-3-yl]-1-benzothiophen-2-carboxamid-Dihydrochlorid in 4 mL THF wird mit 59.6 µL (0.43 mmol) Triethylamin und 50.9 mg (0.43 mmol) Phenylisocyanat versetzt. Nach 16 h bei Raumtemperatur wird das Reaktionsgemisch mittels präparativer HPLC gereinigt. Die eingeengte Produktfraktion wird in einer Mischung aus Acetonitril und 1 N Salzsäure (5:1) gelöst, erneut eingeengt und im Hochvakuum getrocknet. Man erhält 35 mg (35.8 % d. Th.) der Titelverbindung.
¹H-NMR (400 MHz, Methanol-d₄): δ = 8.21 (s, 1H), 8.00 (s, 1H), 7.82 (d, 1H), 7.45 (m, 2H), 7.38 (dd, 1H), 7.30 (m, 2H), 7.03 (dd, 1H), 4.43 (m, 1H), 3.85 (m, 1H), 3.52-3.20 (m, 5H), 2.39 (m, 1H), 2.28 (m, 1H), 2.10 (m, 2H), 1.96 (m, 1H)
HPLC: Rₜ = 3.9 min.
MS (ESIpos): *m*/*z* = 421 (M+H)⁺ (freie Base).

### Beispiel 2

### N-[(3R)-1-Azabicyclo[2.2.2]oct-3-yl]-6-({[(4-chlorphenyl)-amino]-carbonyl}-amino)-1-benzothiophen-2-carboxamid-Hydrochlorid

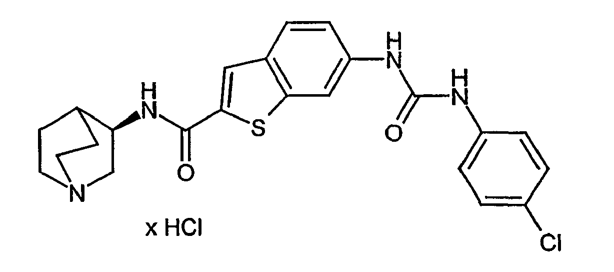

Eine Lösung aus 40 mg (0.11 mmol) 6-Amino-N-[(3R)-1-azabicyclo[2.2.2]oct-3-yl]-1-benzothiophen-2-carboxamid-Dihydrochlorid in 2 mL THF wird mit 29.8 µL (0.21 mmol) Triethylamin und 32.8 mg (0.21 mmol) 4-Chlorphenylisocyanat versetzt. Nach 16 h bei Raumtemperatur wird im Vakuum eingeengt. Der Rückstand wird in einem 1:1-Gemisch aus Wasser und Acetonitril gelöst und mittels präparativer HPLC gereinigt. Die eingeengte Produktfraktion wird in einer Mischung aus Acetonitril und 1 N Salzsäure (5:1) gelöst, erneut eingeengt und im Hochvakuum getrocknet. Man erhält 29 mg (55.2 % d. Th.) der Titelverbindung.
¹H-NMR (400 MHz, Methanol-d₄): δ = 8.21 (s, 1H), 8.02 (s, 1H), 7.82 (d, 1H), 7.47 (m, 2H), 7.38 (dd, 1H), 7.29 (m, 2H), 4.44 (m, 1H), 3.85 (m, 1H), 3.52-3.12 (m, 5H), 2.39 (m, 1H), 2.28 (m, 1H), 2.10 (m, 2H), 1.96 (m, 1H)
HPLC: Rₜ = 4.1 min.
MS (ESIpos): *mlz* = 455 (M+H)⁺ (freie Base).

### Beispiel 3

### N-[(3R)-1-Azabicyclo[2.2.2]oct-3-yl]-6-({[(2-methoxyphenyl)-amino]-carbonyl}-amino)-1-benzothiophen-2-carboxamid-Hydrochlorid

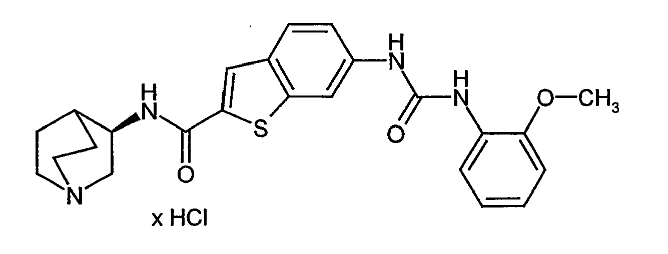

Eine Lösung von 80 mg (0.21 mmol) 6-Amino-N-[(3R)-1-azabicyclo[2.2.2]oct-3-yl]-1-benzothiophen-2-carboxamid-Dihydrochlorid in 4 mL THF wird mit 59.6 µL (0.43 mmol) Triethylamin und 63.8 mg (0.43 mmol) 2-Methoxyphenylisocyanat versetzt. Nach 16 h bei Raumtemperatur wird mittels präparativer HPLC gereinigt. Die eingeengte Produktfraktion wird in einer Mischung aus Acetonitril und 1 N Salzsäure (5:1) gelöst, erneut eingeengt und im Hochvakuum getrocknet. Man erhält 10 mg (9.6 % d. Th.) der Titelverbindung.
¹H-NMR (400 MHz, Methanol-d₄): δ = 8.23 (s, 1H), 8.09 (d, 1H), 8.03 (s, 1H), 7.82 (d, 1H), 7.36 (m, 1H), 7.00 (m, 2H), 6.92 (m, 1H), 4.44 (m, 1H), 3.93 (s, 3H), 3.84 (m, 1H), 3.48 (m, 1H), 3.42-3.25 (m, 4H), 2.39 (m, 1H), 2.28 (m, 1H), 2.10 (m, 2H), 1.96 (m, 1H)
HPLC: Rₜ = 4.0 min.
MS (ESIpos): *m*/*z* = 451 (M+H)⁺ (freie Base).

### Beispiel 4

### N-[(3R)-1-Azabicyclo[2.2.2]oct-3-yl]-6-({[(4-methoxyphenyl)-amino]-carbonyl}-amino)-1-benzothiophen-2-carboxamid-Hydrochloric

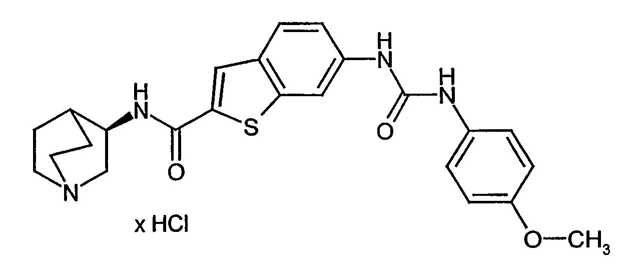

Eine Lösung von 80 mg (0.21 mmol) 6-Amino-N-[(3R)-1-azabicyclo[2.2.2]oct-3-yl]-1-benzothiophen-2-carboxamid-Dihydrochlorid in 4 mL THF wird mit 59.6 µL (0.43 mmol) Triethylamin und 63.8 mg (0.43 mmol) 4-Methoxyphenylisocyanat versetzt. Nach 16 h bei Raumtemperatur wird mittels präparativer HPLC gereinigt. Die eingeengte Produktfraktion wird in einer Mischung aus Acetonitril und 1 N Salzsäure (5:1) gelöst, erneut eingeengt und im Hochvakuum getrocknet. Man erhält 55 mg (52.8 % d. Th.) der Titelverbindung.
¹H-NMR (400 MHz, Methanol-d₄): δ = 8.20 (s, 1H), 8.00 (s, 1H), 7.81 (d, 1H), 7.35 (m, 3H), 6.89 (m, 2H), 4.44 (m, 1H), 3.85 (m, 1H), 3.78 (s, 3H), 3.52-3.22 (m, 5H), 2.39 (m, 1H), 2.28 (m, 1H), 2.10 (m, 2H), 1.96 (m, 1H)
HPLC: Rₜ = 3.8 min.
MS (ESIpos): *m*/*z* = 451 (M+H)⁺ (freie Base).

### Beispiel 5

### N-[(3R)-1-Azabicyclo[2.2.2]oct-3-yl]-6-({[(2-phenylethyl)-amino]-carbonyl}-amino)-1-benzothiophen-2-carboxamid-Hydrochlorid

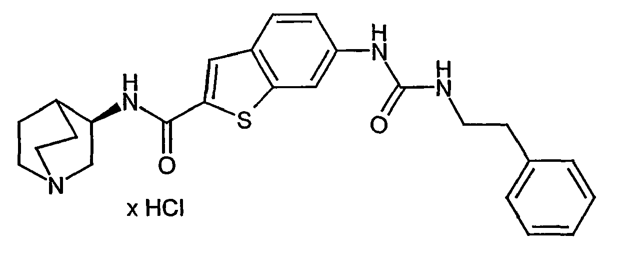

Eine Lösung von 40 mg (0.11 mmol) 6-Amino-N-[(3R)-1-azabicyclo[2.2.2]oct-3-yl]-1-benzothiophen-2-carboxamid-Dihydrochlorid in 2 mL THF wird mit 29.8 µL (0.21 mmol) Triethylamin und 31.5 mg (0.21 mmol) (2-Isocyanatoethyl)-benzol versetzt. Nach 18 h bei Raumtemperatur wird mittels präparativer HPLC gereinigt. Die eingeengte Produktfraktion wird in einer Mischung aus Acetonitril und 1 N Salzsäure (5:1) gelöst, erneut eingeengt und im Hochvakuum getrocknet. Man erhält 32 mg (61.8 % d. Th.) der Titelverbindung.
¹H-NMR (400 MHz, Methanol-d₄): δ = 8.11 (s, 1H), 8.02 (s, 1H), 7.77 (d, 1H), 7.36-7.16 (m, 6H), 4.44 (m, 1H), 3.83 (m, 1H), 3.55-3.23 (m, 7H), 2.86 (tr, 2H), 2.38 (m, 1H), 2.28 (m, 1H), 2.10 (m, 2H), 1.96 (m, 1H)
HPLC: Rₜ = 4.0 min.
MS (ESIpos): *m*/*z* = 449 (M+H)⁺ (freie Base).

### Beispiel 6

### N-[(3R)-1-Azabicyclo[2.2.2]oct-3-yl]-6-({[(3-cyanophenyl)-amino]-carbonyl}-amino)-1-benzothiophen-2-carboxamid-Hydrochlorid

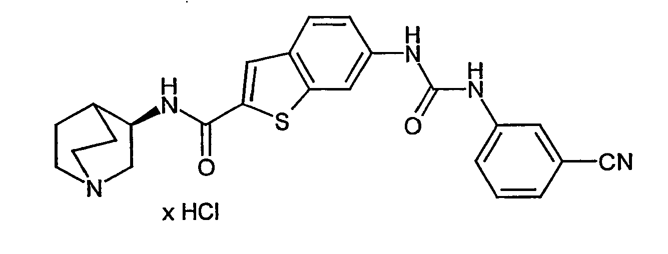

Eine Lösung von 40 mg (0.11 mmol) 6-Amino-N-[(3R)-1-azabicyclo[2.2.2]oct-3-yl]-1-benzothiophen-2-carboxamid-Dihydrochlorid in 2 mL THF wird mit 29.8 µL (0.21 mmol) Triethylamin und 30.8 mg (0.21 mmol) 3-Cyanophenylisocyanat versetzt. Nach 18 h bei Raumtemperatur wird mittels präparativer HPLC gereinigt. Die eingeengte Produktfrakkion wird in einer Mischung aus Acetonitril und 1 N Salzsäure (5:1) gelöst, erneut eingeengt und im Hochvakuum getrocknet. Man erhält 32 mg (62.1 % d. Th.) der Titelverbindung.
¹H-NMR (400 MHz, Methanol-d₄): δ = 8.23 (s, 1H), 8.00 (m, 2H), 7.83 (d, 1H), 7.67 (d, 1H), 7.47 (dd, 1H), 7.39 (m, 2H), 4.43 (m, 1H), 3.86 (m, 1H), 3.53-3.18 (m, 5H), 2.39 (m, 1H), 2.28 (m, 1H), 2.10 (m, 2H), 1.97 (m, 1H).
HPLC: Rₜ = 3.9 min.
MS (ESIpos): *m*/*z* = 446 (M+H)⁺ (freie Base).

### Beispiel 7

### N-[(3R)-1-Azabicyclo[2.2.2]oct-3-yl]-6-({[(3-bromphenyl)-amino]-carbonyl}-amino)-1-benzothiophen-2-carboxamid-Hydrochlorid

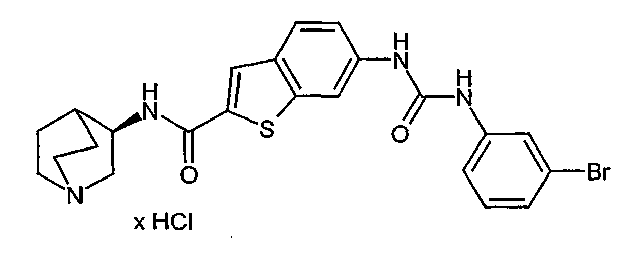

Eine Lösung von 40 mg (0.11 mmol) 6-Amino-N-[(3R)-1-azabicyclo[2.2.2]oct-3-yl]-1-benzothiophen-2-carboxamid-Dihydrochlorid in 2 mL THF wird mit 29.8 µL (0.21 mmol) Triethylamin und 42.3 mg (0.21 mmol) 3-Bromphenylisocyanat versetzt. Nach 18 h bei Raumtemperatur wird mittels präparativer HPLC aufgereinigt. Die eingeengte Produktfraktion wird in einer Mischung aus Acetonitril und 1 N Salzsäure (5:1) gelöst, erneut eingeengt und im Hochvakuum getrocknet. Man erhält 43 mg (75.1 % d. Th.) der Titelverbindung.
¹H-NMR (400 MHz, Methanol-d₄): δ = 8.21 (s, 1H), 8.02 (m, 1H), 7.82 (m, 2H), 7.37 (m, 2H), 7.19 (m, 2H), 4.45 (m, 1H), 3.84 (m, 1H), 3.54-3.20 (m, 5H), 2.39 (m, 1H), 2.28 (m, 1H), 2.11 (m, 2H), 1.97 (m, 1H)
HPLC: Rₜ = 4.2 min.
MS (ESIpos): *m*/*z* = 499 (M+H)⁺ (freie Base).

### Beispiel 8

### N-[(3R)-1-Azabicyclo[2.2.2]oct-3-yl]-6-({[(2-ethoxyphenyl)-amino]-carbonyl}-amino)-1-benzothiophen-2-carboxamid-Hydrochlorid

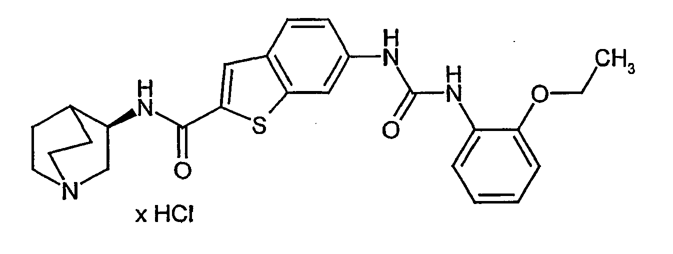

Eine Lösung von 40 mg (0.11 mmol) 6-Amino-N-[(3R)-1-azabicyclo[2.2.2]oct-3-yl]-1-benzothiophen-2-carboxamid-Dihydrochlorid in 2 mL THF wird mit 29.8 µL (0.21 mmol) Triethylamin und 34.9 mg (0.21 mmol) 2-Ethoxyphenylisocyanat versetzt. Nach 18 h bei Raumtemperatur wird mittels präparativer HPLC gereinigt. Die eingeengte Produktfraktion wird in einer Mischung aus Acetonitril und 1 N Salzsäure (5:1) gelöst, erneut eingeengt und im Hochvakuum getrocknet. Man erhält 10 mg (18.7 % d. Th.) der Titelverbindung.
¹H-NMR (400 MHz, Methanol-d₄): δ = 8.25 (s, 1H), 8.09 (d, 1H), 8.00 (s, 1H), 7.82 (d, 1H), 7.38 (d, 1H), 6.98 (m, 2H), 6.91 (m, 1H), 4.43 (m, 1H), 4.16 (q, 2H), 3.86 (m, 1H), 3.53-3.21 (m, 5H), 2.39 (m, 1H), 2.28 (m, 1H), 2.10 (m, 2H), 1.97 (m, 1H), 1.50 (tr, 3H)
HPLC: Rₜ = 4.2 min.
MS (ESIpos): *mlz* = 465 (M+H)⁺ (freie Base).

### Beispiel 9

### N-[(3R)-1-Azabicyclo[2.2.2]oct-3-yl]-6-({ [(4-(dimethylamino)-phenyl)-amino]-carbonyl}-amino)-1-benzothiophen-2-carboxamid-Hydrochlorid

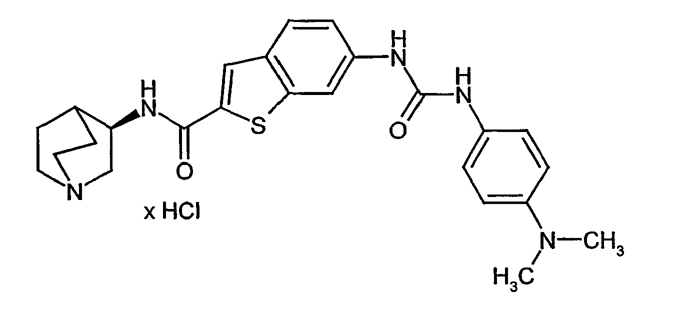

Eine Lösung von 40 mg (0.11 mmol) 6-Amino-N-[(3R)-1-azabicyclo[2.2.2]oct-3-yl]-1-benzothiophen-2-carboxamid-Dihydrochlorid in 2 mL THF wird mit 29.8 µL (0.21 mmol) Triethylamin und 34.7 mg (0.21 mmol) 4-N,N-Dimethylaminophenylisocyanat versetzt. Nach 18 h bei Raumtemperatur wird mittels präparativer HPLC gereinigt. Die eingeengte Produktfraktion wird in einer Mischung aus Acetonitril und 1 N Salzsäure (5:1) gelöst, erneut eingeengt und im Hochvakuum getrocknet. Man erhält 21 mg (38.7 % d. Th.) der Titelverbindung.
¹H-NMR (400 MHz, Methanol-d₄): δ = 8.24 (s, 1H), 8.05 (s, 1H), 7.84 (d, 1H), 7.72 (m, 2H), 7.58 (m, 2H), 7.39 (d, 1H), 4.45 (m, 1H), 3.84 (m, 1H), 3.53-3.20 (m, 11H), 2.39 (m, 1H), 2.28 (m, 1H), 2.11 (m, 2H), 1.97 (m, 1H)
HPLC: Rₜ = 3.3 min.
MS (ESIpos): *m*/*z* = 464 (M+H)⁺ (freie Base).

### Beispiel 10

### N-[(3R)-1-Azabicyclo[2.2.2]oct-3-yl]-6-({[(2-nitrophenyl)-amino]-carbonyl}-amino)-1-benzothiophen-2-carboxamid-Hydrochlorid

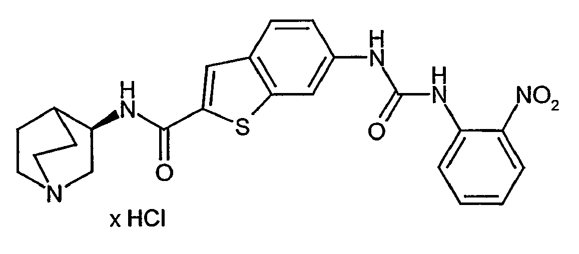

Eine Lösung von 40 mg (0.11 mmol) 6-Amino-N-[(3R)-1-azabicyclo[2.2.2]oct-3-yl]-1-benzothiophen-2-carboxamid-Dihydrochlorid in 2 mL THF wird mit 29.8 µL (0.21 mmol) Triethylamin und 35.1 mg (0.21 mmol) 2-Nitrophenylisocyanat versetzt. Nach 18 h bei Raumtemperatur wird mittels präparativer HPLC gereinigt. Die eingeengte Produktfraktion wird in einer Mischung aus Acetonitril und 1 N Salzsäure (5:1) gelöst, erneut eingeengt und im Hochvakuum getrocknet. Man erhält 6 mg (11.2 % d. Th.) der Titelverbindung.
¹H-NMR (400 MHz, Methanol-d₄): δ = 8.51 (d, 1H), 8.30 (s, 1H), 8.20 (d, 1H), 8.01 (s, 1H), 7.85 (d, 1H), 7.69 (dd, 1H), 7.45 (d, 1H), 7.20 (dd, 1H), 4.45 (m, 1H), 3.86 (m, 1H), 3.52-3.10 (m, 5H), 2.39 (m, 1H), 2.28 (m, 1H), 2.11 (m, 2H), 1.97 (m, 1H)
HPLC: Rₜ = 4.1 min.
MS (ESIpos): *m*/*z* = 466 (M+H)⁺ (freie Base).

### Beispiel 11

### N-[(3R)-1-Azabicyclo[2.2.2]oct-3-yl]-6-({[(2,6-difluorphenyl)-amino]-carbonyl}-amino)-1-benzothiophen-2-carboxamid-Hydrochlorid

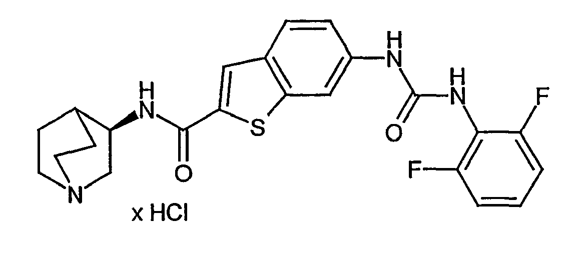

Eine Lösung von 40 mg (0.11 mmol) 6-Amino-N-[(3R)-1-azabicyclo[2.2.2]oct-3-yl]-1-benzothiophen-2-carboxamid-Dihydrochlorid in 2 mL THF wird mit 29.8 µL (0.21 mmol) Triethylamin und 33.2 mg (0.21 mmol) 2,6-Difluorphenylisocyanat versetzt. Nach 18 h bei Raumtemperatur wird mittels präparativer HPLC gereinigt. Die eingeengte Produktfraktion wird in einer Mischung aus Acetonitril und 1 N Salzsäure (5:1) gelöst, erneut eingeengt und im Hochvakuum getrocknet. Man erhält 40 mg (75.9 % d. Th.) der Titelverbindung.
¹H-NMR (400 MHz, Methanol-d₄): δ = 8.18 (s, 1H), 8.01 (s, 1H), 7.83 (d, 1H), 7.41 (d, 1H), 7.30 (m, 1H), 7.06 (m, 2H), 4.43 (m, 1H), 3.85 (m, 1H), 3.52-3.22 (m, 5H), 2.39 (m, 1H), 2.28 (m, 1H), 2.11 (m, 2H), 1.97 (m, 1H)
HPLC: Rₜ = 3.8 min.
MS (ESIpos): *m*/*z* = 457 (M+H)⁺ (freie Base).

### Beispiel 12

### N-[(3R)-1-Azabicyclo[2.2.2]oct-3-yl]-6-({[(2,4-dichlorphenyl)-amino]-carbonyl}-amino)-1-benzothiophen-2-carboxamid-Hydrochlorid

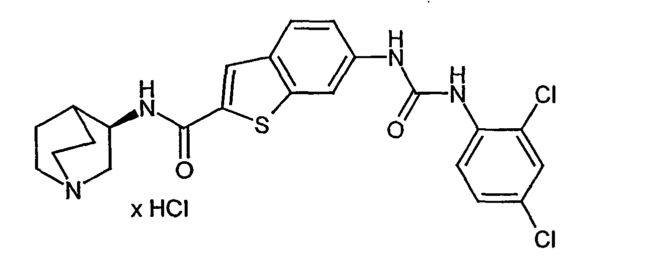

Eine Lösung von 40 mg (0.11 mmol) 6-Amino-N-[(3R)-1-azabicyclo[2.2.2]oct-3-yl]-1-benzothiophen-2-carboxamid-Dihydrochlorid in 2 mL THF wird mit 29.8 µL (0.21 mmol) Triethylamin und 40.2 mg (0.21 mmol) 2,4-Dichlorphenylisocyanat versetzt. Nach 18 h bei Raumtemperatur wird mittels präparativer HPLC gereinigt. Die eingeengte Produktfraktion wird in einer Mischung aus Acetonitril und 1 N Salzsäure (5:1) gelöst, erneut eingeengt und im Hochvakuum getrocknet. Man erhält 25 mg (44.5 % d. Th.) der Titelverbindung.
¹H-NMR (400 MHz, Methanol-d₄): δ = 8.26 (s, 1H), 8.19 (d, 1H), 8.01 (s, 1H), 7.83 (d, 1H), 7.49 (m, 1H), 7.38 (d, 1H), 7.31 (d, 1H), 4.43 (m, 1H), 3.85 (m, 1H), 3.51-3.18 (m, 5H), 2.38 (m, 1H), 2.27 (m, 1H), 2.10 (m, 2H), 1.96 (m, 1H)
HPLC: Rₜ = 4.4 min.
MS (ESIpos): *m*/*z* = 489 (M+H)⁺ (freie Base).

### Beispiel 13

### N-[(3R)-1-Azabicyclo[2.2.2]oct-3-yl]-6-[({[3-(trifluormethyl)-phenyl]-amino}-carbonyl)-amino]-1-benzothiophen-2-carboxamid-Hydrochlorid

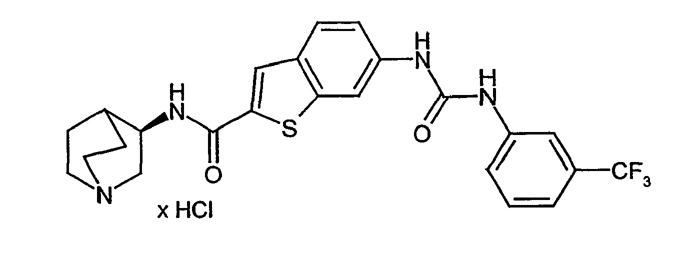

Eine Lösung von 40 mg (0.11 mmol) 6-Amino-N-[(3R)-1-azabicyclo[2.2.2]oct-3-yl]-1-benzothiophen-2-carboxamid-Dihydrochlorid in 2 mL THF wird mit 29.8 µL (0.21 mmol) Triethylamin und 40.0 mg (0.21 mmol) 3-Trifluormethylphenylisocyanat versetzt. Nach 18 h bei Raumtemperatur wird mittels präparativer HPLC gereinigt. Die eingeengte Produktfraktion wird in einer Mischung aus Acetonitril und 1 N Salzsäure (5:1) gelöst, erneut eingeengt und im Hochvakuum getrocknet. Man erhält 51 mg (89 % d. Th.) der Titelverbindung.
¹H-NMR (400 MHz, Methanol-d₄): δ = 8.22 (s, 1H), 8.04 (s, 1H), 7.93 (s, 1H), 7.83 (d, 1H), 7.63 (d, 1H), 7.49 (dd, 1H), 7.40 (m, 1H), 7.31 (d, 1H), 4.44 (m, 1H), 3.84 (m, 1H), 3.54-3.25 (m, 5R), 2.38 (m, 1H), 2.28 (m, 1H), 2.10 (m, 2H), 1.97 (m, 1H)
HPLC: Rₜ = 4.3 min.
MS (ESIpos): *m*/*z* = 489 (M+H)⁺ (freie Base).

### Beispiel 14

### N-[(3R)-1-Azabicyclo[2.2.2]oct-3-yl]-6-({[(3,4,5-trimethoxyphenyl)-amino]-carbonyl}-amino)-1-benzothiophen-2-carboxamid-Hydrochlorid

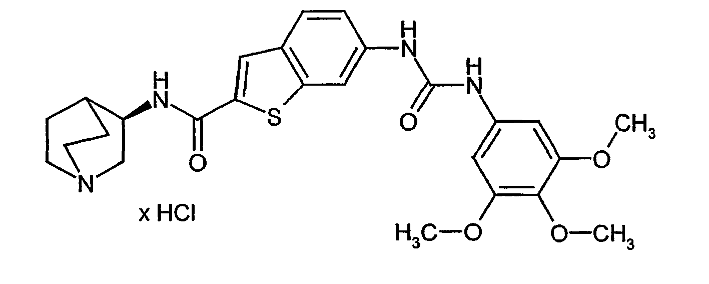

Eine Lösung von 40 mg (0.11 mmol) 6-Amino-N-[(3R)-1-azabicyclo[2.2.2]oct-3-yl]-1-benzothiophen-2-carboxamid-Dihydrochlorid in 2 mL THF wird mit 29.8 µL (0.21 mmol) Triethylamin und 44.7 mg (0.21 mmol) 3,4,5-Trimethoxyphenylisocyanat versetzt. Nach 18 h bei Raumtemperatur wird mittels präparativer HPLC gereinigt. Die eingeengte Produktfraktion wird in einer Mischung aus Acetonitril und 1 N Salzsäure (5:1) gelöst, erneut eingeengt und im Hochvakuum getrocknet. Man erhält 11 mg (17.4 % d. Th.) der Titelverbindung.
¹H-NMR (400 MHz, Methanol-d₄): δ = 8.22 (s, 1H), 8.00 (s, 1H), 7.82 (d, 1H), 7.38 (d, 1H), 6.82 (s, 2H), 4.43 (m, 1H), 3.85 (m, 7H), 3.73 (s, 3H), 3.52-3.18 (m, 5H), 2.39 (m, 1H), 2.28 (m, 1H), 2.10 (m, 2H), 1.96 (m, 1H)
HPLC: Rₜ = 3.8 min.
MS (ESIpos): *m*/*z* = 511 (M+H)⁺ (freie Base).

### Beispiel 15

### N-[(3R)-1-Azabicyclo[2.2.2]oct-3-yl]-6-[({[4-methoxy-3-(trifluormethyl)-phenyl]-amino}-carbonyl)-amino]-1-benzothiophen-2-carboxamid-Hydrochlorid

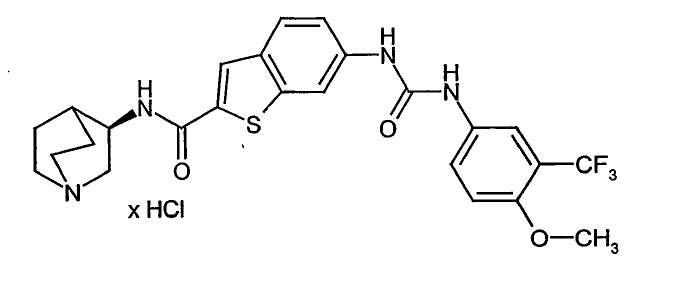

Eine Lösung von 40 mg (0.11 mmol) 6-Amino-N-[(3R)-1-azabicyclo[2.2.2]oct-3-yl]-1-benzothiophen-2-carboxamid-Dihydrochlorid in 2 mL THF und 0.2 mL DMF wird mit 29.8 µL (0.21 mmol) Triethylamin und 46.4 mg (0.21 mmol) 4-Methoxy-3-trifluormethylphenylisocyanat versetzt. Nach 18 h bei Raumtemperatur wird mittels präparativer HPLC gereinigt. Die eingeengte Produktfraktion wird in einer Mischung aus Methanol und 1 N Salzsäure (2:1) gelöst, erneut eingeengt und im Hochvakuum getrocknet. Man erhält 15 mg (25.3 % d. Th.) der Titelverbindung.
¹H-NMR (300 MHz, Methanol-d₄): δ = 8.18 (d, 1H), 8.02 (s, 1H), 7.81 (d, 1H), 7.73 (d, 1H), 7.60 (dd, 1H), 7.37 (dd, 1H), 7.13 (d, 1H), 4.43 (m, 1H), 3.87 (s, 3H), 3.83 (m, 1H), 3.53-3.17 (m, 5H), 2.38 (m, 1H), 2.27 (m, 1H), 2.10 (m, 2H), 1.96 (m, 1H)
HPLC: Rₜ = 4.2 min.
MS (ESIpos): m/z = 519 (M+H)⁺ (freie Base).

### Beispiel 16

### N-[(3R)-1-Azabicyclo[2.2.2]oct-3-yl]-6-[({[3-methoxyphenyl]-amino}-carbonyl)-amino]-1-benzothiophen-2-carboxamid-Hydrochlorid

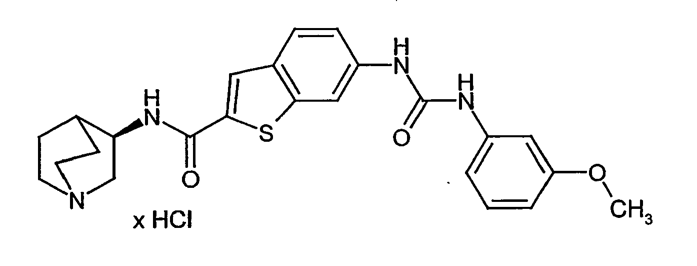

Eine Lösung von 40 mg (0.11 mmol) 6-Amino-N-[(3R)-1-azabicyclo[2.2.2]oct-3-yl]-1-benzothiophen-2-carboxamid-Dihydrochlorid in 2 mL THF und 0.2 mL DMF wird mit 29.8 µL (0.21 mmol) Triethylamin und 31.9 mg (0.21 mmol) 3-Methoxyphenylisocyanat versetzt. Nach 18 h bei Raumtemperatur wird mittels präparativer HPLC gereinigt. Die eingeengte Produktfraktion wird in einer Mischung aus Methanol und 1 N Salzsäure (2:1) gelöst, erneut eingeengt und im Hochvakuum getrocknet. Man erhält 17 mg (32.7 % d. Th.) der Titelverbindung.
¹H-NMR (300 MHz, Methanol-d₄): δ = 8.20 (d, 1H), 8.02 (s, 1H), 7.81 (d, 1H), 7.37 (dd, 1H), 7.08 (m, 2H), 6.93 (m, 1H), 6.61 (m, 1H), 4.43 (m, 1H), 3.83 (m, 1H), 3.79 (s, 3H), 3.53-3.15 (m, 5H), 2.38 (m, 1H), 2.27 (m, 1H), 2.10 (m, 2H), 1.95 (m, 1H)
HPLC: Rₜ = 4.0 min.
MS (ESIpos): *m*/*z* = 451 (M+H)⁺ (freie Base).

### Beispiel 17

### N-[(3R)-1-Azabicyclo[2.2.2]oct-3-yl]-6-[({[3-trifluormethoxyphenyl]-amino}-carbonyl)-amino]-1-benzothiophen-2-carboxamid-Hydrochlorid

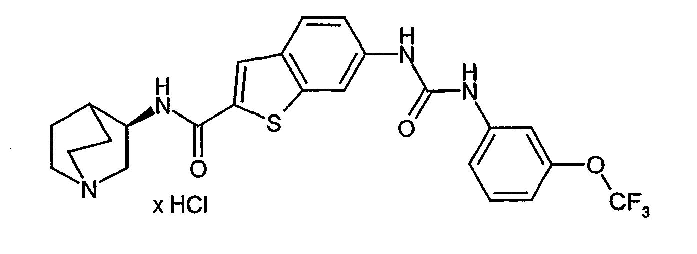

Eine Lösung von 40 mg (0.11 mmol) 6-Amino-N-[(3R)-1-azabicyclo[2.2.2]oct-3-yl]-1-benzothiophen-2-carboxamid-Dihydrochlorid in 2 mL THF und 0.2 mL DMF wird mit 29.8 µL (0.21 mmol) Triethylamin und 43.4 mg (0.21 mmol) 3-Trifluormethoxyphenylisocyanat versetzt. Nach 18 h bei Raumtemperatur wird mittels präparativer HPLC gereinigt. Die eingeengte Produktfraktion wird in einer Mischung aus Methanol und 1 N Salzsäure (2:1) gelöst, erneut eingeengt und im Hochvakuum getrocknet. Man erhält 8.5 mg (14.7 % d. Th.) der Titelverbindung.
¹H-NMR (300 MHz, Methanol-d₄): δ = 8.21 (d, 1H), 8.01 (s, 1H), 7.83 (d, 1H), 7.62 (s, 1H), 7.85 (m, 3H), 6.92 (m, 1H), 4.45 (m, 1H), 3.84 (m, 1H), 3.56-3.06 (m, 5R), 2.38 (m, 1H), 2.28 (m, 1H), 2.10 (m, 2H), 1.96 (m, 1H)
HPLC: Rₜ = 4.4 min.
MS (ESIpos): *m*/*z* = 505 (M+H)⁺ (freie Base).

### Beispiel 18

### N-[(3R)-1-Azabicyclo[2.2.2]oct-3-yl]-6-{[(tert-butylamino)-carbonyl]-amino}-1-benzothiophen-2-carboxamid-Hydrochlorid

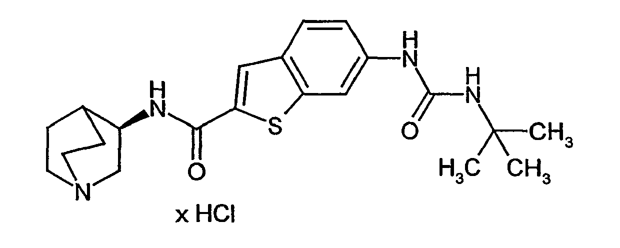

Eine Lösung von 50 mg (0.13 mmol) 6-Amino-N-[(3R)-1-azabicyclo[2.2.2]oct-3-yl]-1-benzothiophen-2-carboxamid-Dihydrochlorid in 2 mL THF und 0.2 mL DMF wird mit 37.2 µL (0.27 mmol) Triethylamin und 26.5 mg (0.27 mmol) tert.-Butylisocyanat versetzt. Nach 18 h bei Raumtemperatur wird mittels präparativer HPLC gereinigt. Die eingeengte Produktfraktion wird in einer Mischung aus Acetonitril und 1 N Salzsäure (5:1) gelöst, erneut eingeengt und im Hochvakuum getrocknet. Man erhält 19 mg (32.6 % d. Th.) der Titelverbindung.
¹H-NMR (400 MHz, Methanol-d₄): δ = 8.08 (m, 1H), 7.98 (s, 1H), 7.75 (d, 1H), 7.23 (dd, 1H), 4.43 (m, 1H), 3.83 (m, 1H), 3.50-3.18 (m, 5H), 2.38 (m, 1H), 2.27 (m, 1H), 2.10 (m, 2H), 1.95 (m, 1H), 1.38 (m, 9H)
HPLC: Rₜ = 3.8 min.
MS (ESIpos): *m*/*z* = 401 (M+H)⁺ (freie Base).

### Beispiel 19

### N-[(3R)-1-Azabicyclo [2.2.2]oct-3-yl]-6-{ [(cyclohexylamino)-carbonyl]-amino}-1-benzothiophen-2-carboxamid-Hydrochlorid

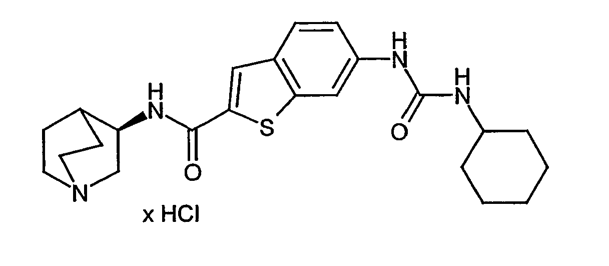

Eine Lösung von 40 mg (0.11 mmol) 6-Amino-N-[(3R)-1-azabicyclo[2.2.2]oct-3-yl]-1-benzothiophen-2-carboxamid-Dihydrochlorid in 2 mL THF und 0.2 mL DMF wird mit 29.8 µL (0.21 mmol) Triethylamin und 26.8 mg (0.21 mmol) Cyclohexylisocyanat versetzt. Nach 18 h bei Raumtemperatur wird mittels präparativer HPLC gereinigt. Die eingeengte Produktfraktion wird in einer Mischung aus Methanol und 1 N Salzsäure (2:1) gelöst, erneut eingeengt und im Hochvakuum getrocknet. Man erhält 23.4 mg (47.3 % d. Th.) der Titelverbindung.
¹H-NMR (300 MHz, Methanol-d₄): δ = 8.10 (d, 1H), 7.99 (s, 1H), 7.77 (d, 1H), 7.27 (dd, 1H), 4.43 (m, 1H), 3.82 (m, 1H), 3.64-3.10 (m, 6H), 2.37 (m, 1H), 2.28 (m, 1H), 2.09 (m, 2H), 1.94 (m, 3H), 1.76 (m, 2H), 1.62 (m, 1H), 1.40 (m, 2H), 1.26 (m, 3H)
HPLC: Rₜ = 4.0 min.
MS (ESIpos): *m*/*z* = 427 (M+H)⁺ (freie Base).

### Beispiel 20

### N-[(3R)-1-Azabicyclo[2.2.2]oct-3-yl]-6-[({[(1S)-1-phenylethyl]-amino}-carbonyl)-amino]-1-benzothiophen-2-carboxamid-Hydrochlorid

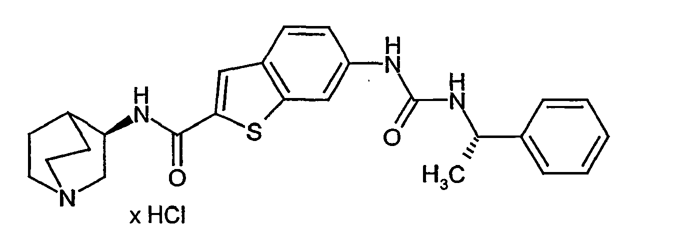

Eine Lösung von 40 mg (0.11 mmol) 6-Amino-N-[(3R)-1-azabicyclo[2.2.2]oct-3-yl]-1-benzothiophen-2-carboxamid-Dihydrochlorid in 2 mL THF und 0.2 mL DMF wird mit 29.8 µL (0.21 mmol) Triethylamin und 31.5 mg (0.21 mmol) (S)-(-)-α-Methylbenzylisocyanat versetzt. Nach 18 h bei Raumtemperatur wird mittels präparativer HPLC gereinigt. Die eingeengte Produktfraktion wird in einer Mischung aus Methanol und 1 N Salzsäure (2:1) gelöst, erneut eingeengt und im Hochvakuum getrocknet. Man erhält 32.5 mg (62.7 % d. Th.) der Titelverbindung.
¹H-NMR (400 MHz, Methanol-d₄): δ = 8.10 (s, 1H), 7.99 (m, 2H), 7.77 (d, 1H), 7.39-7.20 (m, 5H), 4.93 (q, 1H), 4.43 (m, 1H), 3.83 (m, 1H), 3.47 (m, 1H), 3.42-3.26 (m, 4H), 2.37 (m, 1H), 2.27 (m, 1H), 2.09 (m, 2H), 1.95 (m, 1H), 1.49 (d, 3H)
HPLC: Rₜ = 4.0 min.
MS (ESIpos): *m*/*z* = 449 (M+H)⁺ (freie Base).

### Beispiel 21

### 7-[(Anilinocarbonyl)-amino]-N-[(3R)-1-azabicyclo[2.2.2]oct-3-yl]-1-benzothiophen-2-carboxamid-Hydrochlorid

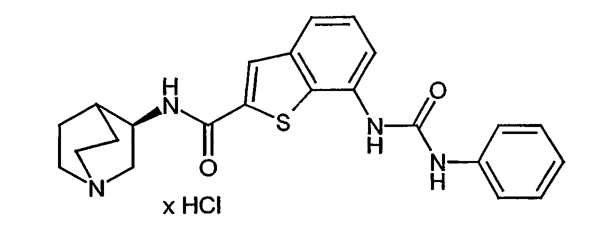

Eine Lösung von 44 mg (0.12 mmol) 7-Amino-N-[(3R)-1-azabicyclo[2.2.2]oct-3-yl]-1-benzothiophen-2-carboxamid-Dihydrochlorid in 2 mL THF wird mit 32.8 µL (0.24 mmol) Triethylamin und 28 mg (0.24 mmol) Phenylisocyanat versetzt. Nach 18 h bei Raumtemperatur wird mittels präparativer HPLC gereinigt. Die eingeengte Produktfraktion wird in einer Mischung aus Acetonitril und 1 N Salzsäure (5:1) gelöst, erneut eingeengt und im Hochvakuum getrocknet. Man erhält 20 mg (36.1 % d. Th.) der Titelverbindung.
¹H-NMR (400 MHz, Methanol-d₄): δ = 8.12 (s, 1H), 7.80 (d, 1H), 7.70 (d, 1H), 7.47 (m, 3H), 7.30 (m, 2H), 7.05 (m, 1H), 4.45 (m, 1H), 3.84 (m, 1H), 3.73 (s, 3H), 3.53-3.21 (m, 5H), 2.39 (m, 1H), 2.28 (m, 1H), 2.10 (m, 2H), 1.96 (m, 1H)
HPLC: Rₜ = 3.9 min.
MS (ESIpos): *m*/*z* = 421 (M+H)⁺ (freie Base).

### Beispiel 22

### N-[(3R)-1-Azabicyclo[2.2.2]oct-3-yl]-6-({[(4-methoxyphenyl)amino]carbonyl}-amino)-1-benzofuran-2-carboxamid-Hydrochlorid

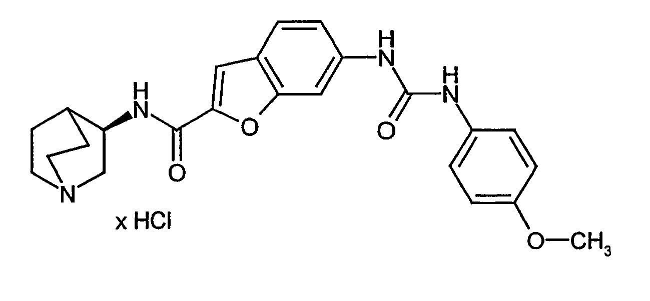

50 mg (0.18 mmol) 6-Amino-N-[(3R)-1-azabicyclo[2.2.2]oct-3-yl]-1-benzofuran-2-carboxamid werden in 3 mL THF/DMF (10:1) vorgelegt und mit 73 µL (0.53 mmol) Triethylamin und 78.4 mg (0.53 mmol) 4-Methoxyphenylisocyanat versetzt. Nach 18 h bei Raumtemperatur und weiteren 20 h bei 50°C wird der Kolbeninhalt im Vakuum eingeengt. Nach dem erneuten Lösen in wenig DMSO und dem Versetzen mit Wasser wird der entstehende Niederschlag abgesaugt. Man löst den Feststoff in DMF/Wasser und reinigt mittels präparativer HPLC. Die eingeengte Produktfraktion wird in einer Mischung aus Methanol und 1 N Salzsäure in Diethylether (5:1) gelöst, erneut eingeengt und im Hochvakuum getrocknet. Man erhält 5.3 mg (7 % d. Th.) der Titelverbindung.
¹H-NMR (300 MHz, Methanol-d₄): δ = 8.07 (s, 1H), 7.60 (m, 1H), 7.47 (s, 1H), 7.33 (m, 2H), 7.12 (dd, 1 H), 6.88 (m, 2H), 4.49 (m, 1 H), 3.82 (m, 1H), 3.77 (s, 3H), 3.54-3.18 (m, 5H), 2.37 (m, 1H), 2.25 (m, 1H), 2.10 (m, 2H), 1.96 (m, 1H)
HPLC: Rₜ = 3.8 min.
MS (ESIpos): *m*/*z* = 435 (M+H)⁺ (freie Base).

## Patentansprüche

1. Verbindungen der Formel 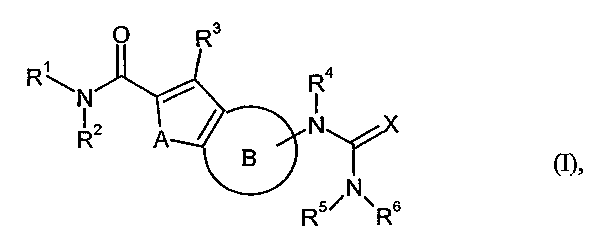 in welcher
R¹ 1-Aza-bicyclo[2.2.2]oct-3-yl,
R² Wasserstoff oder C₁-C₆-Alkyl,
R³ Wasserstoff, Halogen, Amino, Hydroxy oder C₁-C₆-Alkyl,
R⁴ Wasserstoff, C₁-C₆-Alkyl, das gegebenenfalls durch einen Rest ausgewählt aus der Gruppe Hydroxy, Halogen, Cyano, C₁-C₆-Alkoxy, Trifluormethyl, Trifluormethoxy substituiert ist,
R⁵ Wasserstoff oder C₁-C₆-Alkyl, oder
R⁴ und R⁵ zusammen mit den Stickstoffatomen, an die sie gebunden sind, einen 5- bis 6-gliedrigen Heterocyclus, der gegebenenfalls mit bis zu 2 Substituenten unabhängig voneinander ausgewählt aus der Gruppe C₁-C₆-Allcyl, C₁-C₄-Acyl, Oxo, Thioxo substituiert ist,
R⁶ (i) Wasserstoff, (ii) C₁-C₆-Alkyl, (iii) C₃-C₈-Cycloalkyl, (iv) C₆-C₁₀-Aryl, (v) 5- bis 10-gliedriges Heteroaryl, (vi) C₆-C₁₀-Arylcarbonyl wobei (ii) gegebenenfalls mit Phenyl, C₁-C₆-Alkoxycarbonyl oder C₁-C₆-Alkoxy substituiert ist und (iv), (v) und (vi) gegebenenfalls mit bis zu 3 unabhängig voneinander ausgewählten Resten aus der Gruppe C₁-C₆-Alkyl, C₁-C₆-Hydroxyalkyl, 3- bis 8-gliedriges Heterocyclyl, C₆-C₁₀-Aryl, 5- bis 10-gliedriges Heteroaryl, Hydroxy, Halogen, Cyano, C₁-C₆-Alkoxy, C₁-C₆-Acyl, Trifluormethyl, Trifluormethoxy, Nitro, Amino, C₁-C₆-Alkylamino, C₁-C₆-Acylamino substituiert sind, oder
R⁵ und R⁶ zusammen mit dem Stickstoffatom, an das sie gebunden sind, einen 3- bis 10-gliedrigen Heterocyclus, der gegebenenfalls mit C₁-C₆-Alkyl oder C₁-C₆-Hydroxyalkyl substituiert ist,
A Sauerstoff, Stickstoff oder Schwefel,
X Sauerstoff oder Schwefel,
der Ring B Benzo oder Pyrido, die jeweils gegebenenfalls durch Reste aus der Reihe Halogen, Cyano, Trifluormethyl, Trifluormethoxy, Nitro, Amino, C₁-C₆-Alkyl und C₁-C₆-Allcoxy substituiert sind,
bedeuten,
sowie die Solvate, Salze oder Solvate der Salze dieser Verbindungen.

2. Verbindungen nach Anspruch 1, der Formel (I) in welcher
R¹ 1-Aza-bicyclo[2.2.2]oct-3-yl,
R² Wasserstoff oder C₁-C₆-Alkyl,
R³ Wasserstoff, Halogen, Amino, Hydroxy oder C₁-C₆-Alkyl,
R⁴ Wasserstoff, C₁-C₆-Alkyl, das gegebenenfalls durch einen Rest ausgewählt aus der Gruppe Hydroxy, Halogen, Cyano, C₁-C₆-Alkoxy, Trifluormethyl, Trifluormethoxy substituiert ist,
R⁵ Wasserstoff oder C₁-C₆-Alkyl, oder
R⁴ und R⁵ zusammen mit den Stickstoffatomen, an die sie gebunden sind, einen 5- bis 6-gliedrigen Heterocyclus, der gegebenenfalls mit bis zu 2 Substituenten unabhängig voneinander ausgewählt aus der Gruppe C₁-C₆-Alkyl, C₁-C₄-Acyl, Oxo, Thioxo substituiert ist,
R⁶ (i) Wasserstoff, (ii) C₁-C₆-Alkyl, (iii) C₃-C₈-Cycloalkyl, (iv) C₆-C₁₀-Aryl, (v) 5- bis 10-gliedriges Heteroaryl, wobei (ii) gegebenenfalls mit Phenyl oder C₁-C₆-Alkoxy substituiert ist und (iv) und (v) gegebenenfalls mit bis zu 3 unabhängig voneinander ausgewählten Resten aus der Gruppe C₁-C₆-Alkyl, C₁-C₆-Hydroxyalkyl, 3- bis 8-gliedriges Heterocyclyl, C₆-C₁₀-Aryl, 5- bis 10-gliedriges Heteroaryl, Hydroxy, Halogen, Cyano, C₁-C₆-Allcoxy, C₁-C₆-Acyl, Trifluormethyl, Trifluormethoxy, Nitro, Amino, C₁-C₆-Alkylamino, C₁-C₆-Acylamino substituiert sind, oder
R⁵ und R⁶ zusammen mit dem Stickstoffatom, an das sie gebunden sind, einen 3- bis 8-gliedrigen Heterocyclus, der gegebenenfalls mit C₁-C₆-Alkyl oder C₁-C₆-Hydroxyalkyl substituiert ist,
A Sauerstoff, Stickstoff oder Schwefel,
X Sauerstoff oder Schwefel und
der Ring B Benzo oder Pyrido, die jeweils gegebenenfalls durch Reste aus der Reihe Halogen, Cyano, Trifluormethyl, Trifluormethoxy, Nitro, Amino, C₁-C₆-Alkyl und C₁-C₆-Alkoxy substituiert sind,
bedeuten,
sowie die Solvate, Salze oder Solvate der Salze dieser Verbindungen.

3. Verbindungen nach einem der Ansprüche 1 und 2, der Formel (I), in welcher
R¹ 1-Aza-bicyclo[2.2.2]oct-3-yl,
R² Wasserstoff oder C₁-C₄-Alkyl,
R³ Wasserstoff, Halogen, Amino, Hydroxy oder C₁-C₄-Alkyl,
R⁴ Wasserstoff, C₁-C₄-Alkyl, das gegebenenfalls durch einen Rest ausgewählt aus der Gruppe Hydroxy, Halogen, Cyano, C₁-C₃-Alkoxy, Trifluormethyl, Trifluormethoxy substituiert ist,
R⁵ Wasserstoff oder C₁-C₄-Alkyl, oder
R⁴ und R⁵ zusammen mit den Stickstoffatomen, an die sie gebunden sind, einen 5- bis 6-gliedrigen Heterocyclus, der gegebenenfalls mit bis zu 2 Substituenten unabhängig voneinander ausgewählt aus der Gruppe C₁-C₆-Alkyl, C₁-C₄-Acyl, Oxo, Thioxo substituiert ist,
R⁶ (i) Wasserstoff, (ii) C₁-C₄-Alkyl, (iii) C₅-C₆-Cycloalkyl, (iv) Phenyl, (v) 5- bis 6-gliedriges Heteroaryl, (vi) C₆-C₁₀-Arylcarbonyl wobei (ii) gegebenenfalls mit Phenyl, C₁-C₄-Alkoxycarbonyl oder C₁-C₃-Alkoxy substituiert ist und (iv), (v) und (vi) gegebenenfalls mit bis zu 3 unabhängig voneinander ausgewählten Resten aus der Gruppe C₁-C₄-Alkyl, C₁-C₄-Hydroxyalkyl, 3- bis 8-gliedriges Heterocyclyl, C₆-C₁₀-Aryl, 5- bis 10-gliedriges Heteroaryl, Hydroxy, Fluor, Chlor, Cyano, C₁-C₃-Alkoxy, C₁-C₃-Acyl, Trifluormethyl, Trifluormethoxy, Nitro, Amino, C₁-C₃-Alkylamino, C₁-C₃-Acylamino substituiert sind, oder
R⁵ und R⁶ zusammen mit dem Stickstoffatom, an das sie gebunden sind, einen 3- bis 10-gliedrigen Heterocyclus, der gegebenenfalls mit C₁-C₃-Alkyl oder C₁-C₃-Hydroxyalkyl substituiert ist,
A Sauerstoff oder Schwefel,
X Sauerstoff,
der Ring B Benzo oder Pyrido, die jeweils gegebenenfalls durch Reste aus der Reihe Chlor, Fluor, Cyano, Trifluormethyl, Trifluormethoxy, Amino, C₁-C₄-Alkyl und C₁-C₄-Alkoxy substituiert sind,
bedeuten,
sowie die Solvate, Salze oder Solvate der Salze dieser Verbindungen.

4. Verbindungen nach einem der Ansprüche 1 bis 3, der Fomel (I), in welcher
R¹ 1-Aza-bicyclo[2.2.2]oct-3-yl,
R² Wasserstoff oder C₁-C₄-Alkyl,
R³ Wasserstoff, Halogen, Amino, Hydroxy oder C₁-C₄-Alkyl,
R⁴ Wasserstoff oder C₁-C₄-Alkyl, das gegebenenfalls durch einen Rest ausgewählt aus der Gruppe Hydroxy, C₁-C₃-Alkoxy, Trifluormethyl, Trifluormethoxy substituiert ist,
R⁵ Wasserstoff oder C₁-C₄-Alkyl, oder
R⁴ und R⁵ zusammen mit den Stickstoffatomen, an die sie gebunden sind, einen 5- bis 6-gliedrigen Heterocyclus, der gegebenenfalls mit bis zu 2 Substituenten unabhängig voneinander ausgewählt aus der Gruppe C₁-C₆-Alkyl, C₁-C₄-Acyl, Oxo, Thioxo substituiert ist,
R⁶ (i) Wasserstoff, (ii) C₁-C₄-Alkyl, (iii) C₅-C₆-Cycloalkyl, (iv) Phenyl, (v) 5- bis 6-gliedriges Heteroaryl, wobei (ii) gegebenenfalls mit Phenyl substituiert ist und (iv) und (v) gegebenenfalls mit bis zu 3 unabhängig voneinander ausgewählten Resten aus der Gruppe C₁-C₄-Alkyl, C₁-C₄-Hydroxyalkyl, Hydroxy, Chlor, Fluor, Cyano, C₁-C₃-Alkoxy, C₁-C₆-Acyl, Trifluormethyl, Trifluormethoxy, Amino, C₁-C₃-Alkylamino, C₁-C₃-Acylamino substituiert sind, oder
R⁵ und R⁶ zusammen mit dem Stickstoffatom, an das sie gebunden sind, einen 5- bis 6-gliedrigen Heterocyclus, der gegebenenfalls mit C₁-C₃-Alkyl oder C₁-C₃-Hydroxyalkyl substituiert ist,
A Sauerstoff, Stickstoff oder Schwefel,
X Sauerstoff und
der Ring B Benzo oder Pyrido, die jeweils gegebenenfalls durch Reste aus der Reihe Chlor, Fluor, Cyano, Trifluormethyl, Trifluormethoxy, Amino, C₁-C₄-Alkyl und C₁-C₄-Alkoxy substituiert sind,
bedeuten,
sowie die Solvate, Salze oder Solvate der Salze dieser Verbindungen.

5. Verbindungen nach einem der Ansprüche 1 bis 4, der Formel (I), in welcher
R¹ 1-Aza-bicyclo[2.2.2]oct-3-yl,
R² bis R⁴ Wasserstoff,
R⁵ Wasserstoff oder C₁-C₄-Alkyl, oder
R⁴ und R⁵ zusammen mit den Stickstoffatomen, an die sie gebunden sind, einen 5- bis 6-gliedrigen Heterocyclus, der gegebenenfalls mit bis zu 2 Substituenten unabhängig voneinander ausgewählt aus der Gruppe C₁-C₄-Alkyl, C₁-C₄-Acyl, Oxo, Thioxo substituiert ist,
R⁶ (i) Wasserstoff, (ii) C₁-C₄-Alkyl, (iii) C₅-C₆-Cycloalkyl, (iv) Phenyl, (v) Pyridyl, (vi) C₆-C₁₀-Arylcarbonyl wobei (ii) gegebenenfalls mit Phenyl, C₁-C₄-Alkoxycarbonyl oder C₁-C₃-Alkoxy substituiert ist und (iv), (v) und (vi) gegebenenfalls mit bis zu 3 unabhängig voneinander ausgewählten Resten aus der Gruppe C₁-C₄-Alkyl, C₁-C₄-Hydroxyalkyl, 3- bis 8-gliedriges Heterocyclyl, C₆-C₁₀-Aryl, 5- bis 10-gliedriges Heteroaryl, Hydroxy, Fluor, Chlor, Cyano, C₁-C₃-Allcoxy, C₁-C₃-Acyl, Trifluormethyl, Trifluormethoxy, Nitro, Amino, C₁-C₃-Alkylamino, C₁-C₃-Acylamino substituiert sind, oder
R⁵ und R⁶ zusammen mit dem Stickstoffatom, an das sie gebunden sind, einen 3- bis 10-gliedrigen Heterocyclus, der gegebenenfalls mit C₁-C₃-Alkyl oder C₁-C₃-Hydroxyalkyl substituiert ist,
A Sauerstoff oder Schwefel,
X Sauerstoff,
der Ring B Benzo,
bedeuten,
sowie die Solvate, Salze oder Solvate der Salze dieser Verbindungen.

6. Verbindungen nach einem der Ansprüche 1 bis 5, der Fomel (I), in welcher
R¹ 1-Aza-bicyclo[2.2.2]oct-3-yl,
R² Wasserstoff,
R³ Wasserstoff, Chlor, Fluor, Amino oder C₁-C₃-Alkyl,
R⁴ Wasserstoff, Methyl oder Ethyl, wobei Methyl und Ethyl gegebenenfalls durch einen Rest ausgewählt aus der Gruppe Hydroxy, Methoxy, Ethoxy, Trifluormethyl, Trifluormethoxy substituiert sind, oder
R⁴ und R⁵ zusammen mit den Stickstoffatomen, an die sie gebunden sind, einen 5- bis 6-gliedrigen Heterocyclus, der gegebenenfalls mit bis zu 2 Substituenten unabhängig voneinander ausgewählt aus der Gruppe C₁-C₃-Alkyl, C₁-C₄-Acyl, Oxo, Thioxo substituiert ist,
R⁵ Wasserstoff oder C₁-C₃-Alkyl,
R⁶ (i) Wasserstoff, (ii) C₁-C₄-Alkyl, (iii) Cyclopentyl, Cyclohexyl, (iv) Phenyl, (v) Benzyl, (vi) Phenethyl, wobei (iv) bis (vi) gegebenenfalls mit bis zu 3 unabhängig voneinander ausgewählten Resten aus der Gruppe Hydroxy, Chlor, Fluor, Cyano, Methoxy, Ethoxy, C₁-C₄-Acyl, Trifluormethyl, Trifluormethoxy, Amino, C₁-C₃-Alkylamino substituiert sind,
A Sauerstoff oder Schwefel,
X Sauerstoff und
der Ring B Benzo, das gegebenenfalls durch Reste aus der Reihe Chlor, Fluor, Cyano, Trifluormethyl, Trifluormethoxy, C₁-C₄-Alkyl, Methoxy und Ethoxy substituiert ist,
bedeuten, sowie die Solvate, Salze oder Solvate der Salze dieser Verbindungen.

7. Verbindungen der Formel 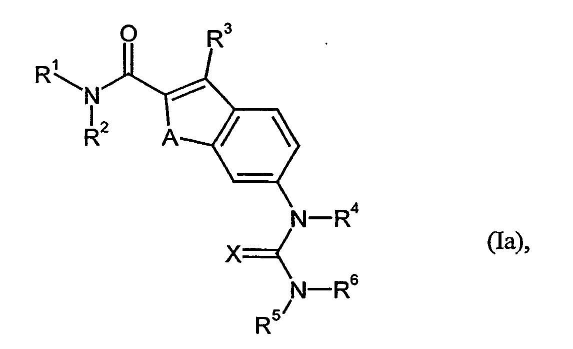 in welcher
R¹ bis R⁶, A und X die in den Ansprüchen 1 bis 6 angegebenen Bedeutungen besitzen, sowie die Solvate, Salze oder Solvate der Salze dieser Verbindungen.

8. Verbindungen der Formel 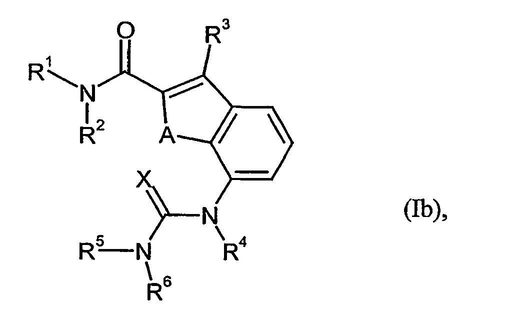 in welcher
R¹ bis R⁶, A und X die in den Ansprüchen 1 bis 6 angegebenen Bedeutungen besitzen, sowie die Solvate, Salze oder Solvate der Salze dieser Verbindungen.

9. Verbindungen der Formel 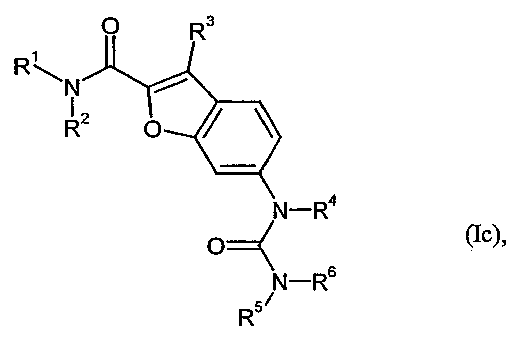 in welcher
R¹ bis R⁶ die in den Ansprüchen 1 bis 6 angegebenen Bedeutungen besitzen, sowie die Solvate, Salze oder Solvate der Salze dieser Verbindungen.

10. Verbindungen der Formel 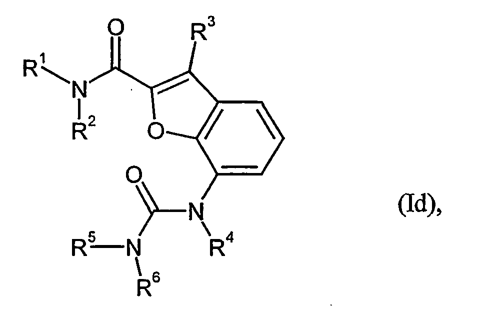 in welcher
R¹ bis R⁶ die in den Ansprüchen 1 bis 6 angegebenen Bedeutungen besitzen, sowie die Solvate, Salze oder Solvate der Salze dieser Verbindungen.

11. Verfahren zur Herstellung von Verbindungen nach Ansprüchen 1 bis 10, wonach man Verbindungen der Formel 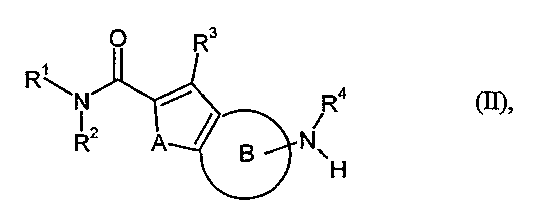 in welcher
R¹ bis R⁴, A und B die in den Ansprüchen 1 bis 10 genannten Bedeutungen haben,
mit Verbindungen der Formel 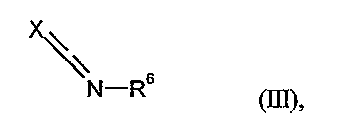 in welcher
X und R⁶ die in den Ansprüchen 1 bis 5 genannten Bedeutungen besitzen,
und die resultierenden Verbindungen (I) gegebenenfalls mit den entsprechenden (a) Lösungsmitteln und/oder (b) Basen oder Säuren zu ihren Solvaten, Salzen oder Solvaten der Salze umsetzt.

12. Verbindungen nach einem der Ansprüche 1 bis 10 zur Behandlung und/oder Prophylaxe von Krankheiten.

13. Arzneimittel enthaltend mindestens eine Verbindung nach einem der Ansprüche 1 bis 10 und mindestens einen pharmazeutisch verträglichen, im wesentlichen nichtgiftigen Träger oder Exzipienten.

14. Verwendung von Verbindungen nach einem der Ansprüche 1 bis 10 zur Herstellung eines Mittels zur Verbesserung der Wahrnehmung, Konzentrationsleistung, Lernleistung und/oder Gedächtnisleistung.

15. Verwendung von Verbindungen nach einem der Ansprüche 1 bis 10 zur Herstellung eines Arzneimittels zur Behandlung und/oder Prophylaxe von Störungen der Wahrnehmung, Konzentrationsleistung, Lernleistung und/oder Gedächtnisleistung.

16. Arzneimittel nach Anspruch 13 zur Behandlung und/oder Prophylaxe von Störungen der Wahrnehmung, Konzentrationsleistung, Lernleistung und/oder Gedächtnisleistung.

## Claims

1. Compounds of the formula 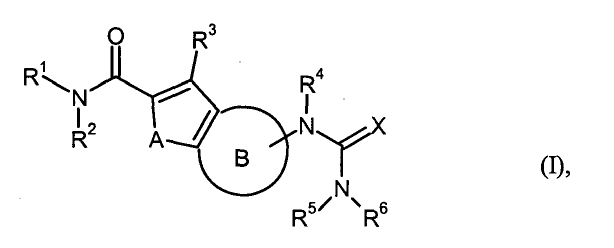 in which
R¹ is 1-azabicyclo[2.2.2]oct-3-yl,
R² is hydrogen or C₁-C₆-alkyl,
R³ is hydrogen, halogen, amino, hydroxy or C₁-C₆-alkyl,
R⁴ is hydrogen, C₁-C₆-alkyl which is optionally substituted by a radical selected from the group of hydroxy, halogen, cyano, C₁-C₆-alkoxy, trifluoromethyl, trifluoromethoxy,
R⁵ is hydrogen or C₁-C₆-alkyl, or
R⁴ and R⁵ together with the nitrogen atom to which they are bonded are a 5- to 6-membered heterocycle which is optionally substituted by up to 2 substituents independently of one another selected from the group of C₁-C₆-alkyl, C₁-C₄-acyl, oxo, thioxo,
R⁶ is (i) hydrogen, (ii) C₁-C₆-alkyl, (iii) C₃-C₈-cycloalkyl, (iv) C₆-C₁₀-aryl, (v) 5- to 10-membered heteroaryl, (vi) C₆-C₁₀-arylcarbonyl, where (ii) is optionally substituted by phenyl, C₁-C₆-alkoxycarbonyl or C₁-C₆-alkoxy, and (iv), (v) and (vi) are optionally substituted by up to 3 radicals selected independently of one another from the group of C₁-C₆-alkyl, C₁-C₆-hydroxyalkyl, 3- to 8-membered heterocyclyl, C₆-C₁₀-aryl, 5- to 10-membered heteroaryl, hydroxy, halogen, cyano, C₁-C₆-alkoxy, C₁-C₆-acyl, trifluoromethyl, trifluoromethoxy, nitro, amino, C₁-C₆-alkylamino, C₁-C₆-acylamino, or
R⁵ and R⁶ together with the nitrogen atom to which they are bonded are a 3- to 10-membered heterocycle which is optionally substituted by C₁-C₆-alkyl or C₁-C₆-hydroxyalkyl,
A is oxygen, nitrogen or sulfur,
X is oxygen or sulfur,
the ring B is benzo or pyrido, each of which are optionally substituted by radicals from the series halogen, cyano, trifluoromethyl, trifluoromethoxy, nitro, amino, C₁-C₆-alkyl and C₁-C₆-alkoxy,
and the solvates, salts or solvates of the salts of these compounds.

2. Compounds according to Claim 1, of the formula (I) in which
R¹ is 1-azabicyclo[2.2.2]oct-3-yl,
R² is hydrogen or C₁-C₆-alkyl,
R³ is hydrogen, halogen, amino, hydroxy or C₁-C₆-alkyl,
R⁴ is hydrogen, C₁-C₆-alkyl which is optionally substituted by a radical selected from the group of hydroxy, halogen, cyano, C₁-C₆-alkoxy, trifluoromethyl, trifluoromethoxy,
R⁵ is hydrogen or C₁-C₆-alkyl, or
R⁴ and R⁵ together with the nitrogen atom to which they are bonded are a 5- to 6-membered heterocycle which is optionally substituted by up to 2 substituents independently of one another selected from the group of C₁-C₆-alkyl, C₁-C₄-acyl, oxo, thioxo,
R⁶ is (i) hydrogen, (ii) C₁-C₆-alkyl, (iii) C₃-C₈-cycloalkyl, (iv) C₆-C₁₀-aryl, (v) 5- to 10-membered heteroaryl, where (ii) is optionally substituted by phenyl, or C₁-C₆-alkoxy, and (iv) and (v) are optionally substituted by up to 3 radicals selected independently of one another from the group of C₁-C₆-alkyl, C₁-C₆-hydroxyalkyl, 3- to 8-membered heterocyclyl, C₆-C₁₀-aryl, 5- to 10-membered heteroaryl, hydroxy, halogen, cyano, C₁-C₆-alkoxy, C₁-C₆-acyl, trifluoromethyl, trifluoromethoxy, nitro, amino, C₁-C₆-alkylamino, C₁-C₆-acylamino, or
R⁵ and R⁶ together with the nitrogen atom to which they are bonded are a 3- to 8-membered heterocycle which is optionally substituted by C₁-C₆-alkyl or C₁-C₆-hydroxyalkyl,
A is oxygen, nitrogen or sulfur, and
X is oxygen or sulfur, and
the ring B is benzo or pyrido, each of which are optionally substituted by radicals from the series halogen, cyano, trifluoromethyl, trifluoromethoxy, nitro, amino, C₁-C₆-alkyl and C₁-C₆-alkoxy,
and the solvates, salts or solvates of the salts of these compounds.

3. Compounds according to either of Claims 1 and 2, of the formula (I) in which
R¹ is 1-aza-bicyclo[2.2.2]oct-3-yl,
R² is hydrogen or C₁-C₄-alkyl,
R³ is hydrogen, halogen, amino, hydroxy or C₁-C₄-alkyl,
R⁴ is hydrogen, C₁-C₄-alkyl which is optionally substituted by a radical selected from the group of hydroxy, halogen, cyano, C₁-C₃-alkoxy, trifluoromethyl, trifluoromethoxy,
R⁵ is hydrogen or C₁-C₄-alkyl, or
R⁴ and R⁵ together with the nitrogen atom to which they are bonded are a 5- to 6-membered heterocycle which is optionally substituted by up to 2 substituents independently of one another selected from the group of C₁-C₆-alkyl, C₁-C₄-acyl, oxo, thioxo,
R⁶ is (i) hydrogen, (ii) C₁-C₄-alkyl, (iii) C₅-C₆-cycloalkyl, (iv) phenyl, (v) 5- to 6-membered heteroaryl, (vi) C₆-C₁₀-arylcarbonyl, where (ii) is optionally substituted by phenyl, C₁-C₄-alkoxycarbonyl or C₁-C₃-alkoxy, and (iv), (v) and (vi) are optionally substituted by up to 3 radicals selected independently of one another from the group of C₁-C₄-alkyl, C₁-C₄-hydroxyalkyl, 3- to 8-membered heterocyclyl, C₆-C₁₀-aryl, 5- to 10-membered heteroaryl, hydroxy, fluorine, chlorine, cyano, C₁-C₃-alkoxy, C₁-C₃-acyl, trifluoromethyl, trifluoromethoxy, nitro, amino, C₁-C₃-alkylamino, C₁-C₃-acylamino, or
R⁵ and R⁶ together with the nitrogen atom to which they are bonded are a 3- to 10-membered heterocycle which is optionally substituted by C₁-C₃-alkyl or C₁-C₃-hydroxyalkyl,
A is oxygen or sulfur,
X is oxygen,
the ring B is benzo or pyrido, each of which are optionally substituted by radicals from the series chlorine, fluorine, cyano, trifluoromethyl, trifluoromethoxy, amino, C₁-C₄-alkyl and C₁-C₄-alkoxy,
and the solvates, salts or solvates of the salts of these compounds.

4. Compounds according to any of Claims 1 to 3, of the formula (I) in which
R¹ is 1-azabicyclo[2.2.2]oct-3-yl,
R² is hydrogen or C₁-C₄-alkyl,
R³ is hydrogen, halogen, amino, hydroxy or C₁-C₄-alkyl,
R⁴ is hydrogen or C₁-C₄-alkyl which is optionally substituted by a radical selected from the group of hydroxy, C₁-C₃-alkoxy, trifluoromethyl, trifluoromethoxy,
R⁵ is hydrogen or C₁-C₄-alkyl, or
R⁴ and R⁵ together with the nitrogen atom to which they are bonded are a 5- to 6-membered heterocycle which is optionally substituted by up to 2 substituents independently of one another selected from the group of C₁-C₆-alkyl, C₁-C₄-acyl, oxo, thioxo,
R⁶ is (i) hydrogen, (ii) C₁-C₄-alkyl, (iii) C₅-C₆-cycloalkyl, (iv) phenyl, (v) 5- to 6-membered heteroaryl, where (ii) is optionally substituted by phenyl, and (iv) and (v) are optionally substituted by up to 3 radicals selected independently of one another from the group of C₁-C₄-alkyl, C₁-C₄-hydroxyalkyl, hydroxy, chlorine, fluorine, cyano, C₁-C₃-alkoxy, C₁-C₆-acyl, trifluoromethyl, trifluoromethoxy, amino, C₁-C₃-alkylamino, C₁-C₃-acylamino, or
R⁵ and R⁶ together with the nitrogen atom to which they are bonded are a 5- to 6-membered heterocycle which is optionally substituted by C₁-C₃-alkyl or C₁-C₃-hydroxyalkyl,
A is oxygen, nitrogen or sulfur,
X is oxygen and
the ring B is benzo or pyrido, each of which are optionally substituted by radicals from the series chlorine, fluorine, cyano, trifluoromethyl, trifluoromethoxy, amino, C₁-C₄-alkyl and C₁-C₄-alkoxy,
and the solvates, salts or solvates of the salts of these compounds.

5. Compounds according to any of Claims 1 to 4, of the formula (I) in which
R¹ is 1-azabicyclo[2.2.2]oct-3-yl,
R² to R⁴ are hydrogen,
R⁵ is hydrogen or C₁-C₄-alkyl, or
R⁴ and R⁵ together with the nitrogen atom to which they are bonded are a 5- to 6-membered heterocycle which is optionally substituted by up to 2 substituents independently of one another selected from the group of C₁-C₄-alkyl, C₁-C₄-acyl, oxo, thioxo,
R⁶ is (i) hydrogen, (ii) C₁-C₄-alkyl, (iii) C₅-C₆-cycloalkyl, (iv) phenyl, (v) pyridyl, (vi) C₆-C₁₀-arylcarbonyl, where (ii) is optionally substituted by phenyl, C₁-C₄-alkoxycarbonyl or C₁-C₃-alkoxy, and (iv), (v) and (vi) are optionally substituted by up to 3 radicals selected independently of one another from the group of C₁-C₄-alkyl, C₁-C₄-hydroxyalkyl, 3- to 8-membered heterocyclyl, C₆-C₁₀-aryl, 5- to 10-membered heteroaryl, hydroxy, fluorine, chlorine, cyano, C₁-C₃-alkoxy, C₁-C₃-acyl, trifluoromethyl, trifluoromethoxy, nitro, amino, C₁-C₃-alkylamino, C₁-C₃-acylamino, or
R⁵ and R⁶ together with the nitrogen atom to which they are bonded are a 3- to 10-membered heterocycle which is optionally substituted by C₁-C₃-alkyl or C₁-C₃-hydroxyalkyl,
A is oxygen or sulfur,
X is oxygen,
the ring B is benzo,
and the solvates, salts or solvates of the salts of these compounds.

6. Compounds according to any of Claims 1 to 5, of the formula (I) in which
R¹ is 1-azabicyclo[2.2.2]oct-3-yl,
R² is hydrogen,
R³ is hydrogen, chlorine, fluorine, amino or C₁-C₃-alkyl,
R⁴ is hydrogen, methyl or ethyl, where methyl and ethyl are optionally substituted by a radical selected from the group of hydroxy, methoxy, ethoxy, trifluoromethyl, trifluoromethoxy, or
R⁴ and R⁵ together with the nitrogen atom to which they are bonded are a 5- to 6-membered heterocycle which is optionally substituted by up to 2 substituents independently of one another selected from the group of C₁-C₃-alkyl, C₁-C₄-acyl, oxo, thioxo,
R⁵ is hydrogen or C₁-C₃-alkyl,
R⁶ is (i) hydrogen, (ii) C₁-C₄-alkyl, (iii) cyclopentyl, cyclohexyl, (iv) phenyl, (v) benzyl, (vi) phenethyl, where (iv) to (vi) are optionally substituted by up to 3 radicals selected independently of one another from the group of hydroxy, chlorine, fluorine, cyano, methoxy, ethoxy, C₁-C₄-acyl, trifluoromethyl, trifluoromethoxy, amino, C₁-C₃-alkylamino,
A is oxygen or sulfur,
X is oxygen and
the ring B is benzo which is optionally substituted by radicals from the series chlorine, fluorine, cyano, trifluoromethyl, trifluoromethoxy, C₁-C₄-alkyl, methoxy and ethoxy,
and the solvates, salts or solvates of the salts of these compounds.

7. Compounds of the formula 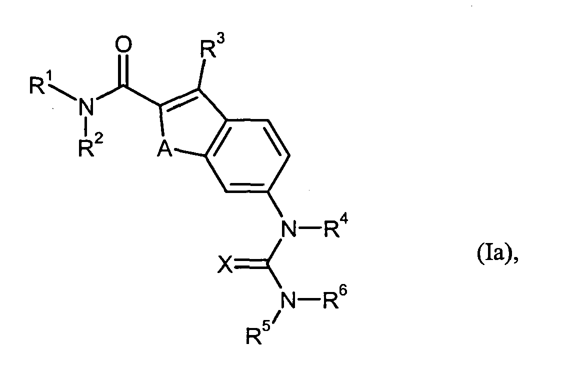 in which
R¹ to R⁶, A and X have the meanings indicated in Claims 1 to 6, and the solvates, salts or solvates of the salts of these compounds.

8. Compounds of the formula 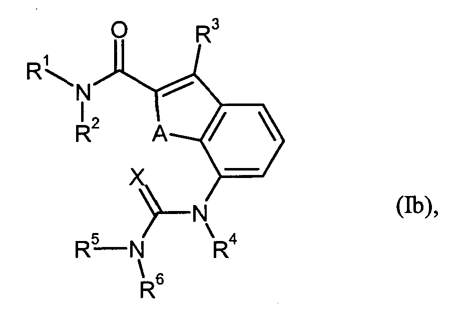 in which
R¹ to R⁶, A and X have the meanings indicated in Claims 1 to 6, and the solvates, salts or solvates of the salts of these compounds.

9. Compounds of the formula 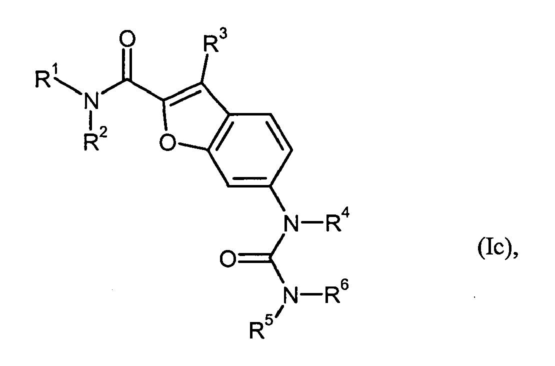 in which
R¹ to R⁶ have the meanings indicated in Claims 1 to 6, and the solvates, salts or solvates of the salts of these compounds.

10. Compounds of the formula 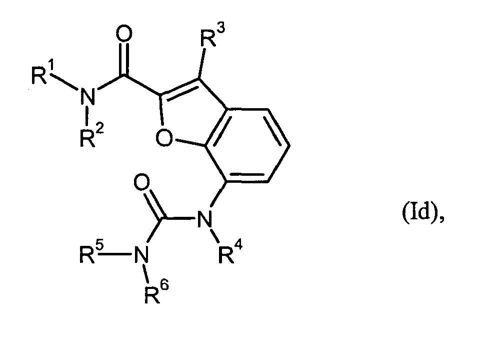 in which
R¹ to R⁶ have the meanings indicated in Claims 1 to 6, and the solvates, salts or solvates of the salts of these compounds.

11. Process for preparing compounds according to Claims 1 to 10, in which compounds of the formula 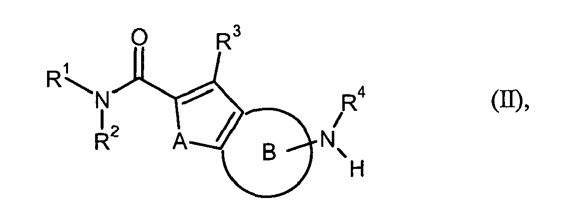 in which
R¹ to R⁴, A and B have the meanings mentioned in Claims 1 to 10,
are reacted with compounds of the formula 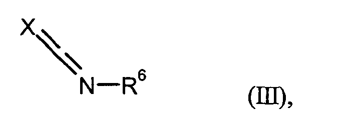 in which
X and R⁶ have the meanings mentioned in Claims 1 to 5,
and the resulting compounds (I) are reacted where appropriate with the appropriate (a) solvents and/or (b) bases or acids to give the solvates, salts or solvates of the salts thereof.

12. Compounds according to any of Claims 1 to 10 for the treatment and/or prophylaxis of diseases.

13. Medicament comprising at least one compound according to any of Claims 1 to 10 and at least one pharmaceutically acceptable, essentially nontoxic carrier or excipient.

14. The use of compounds according to any of Claims 1 to 10 for producing a composition for improving perception, concentration, learning and/or memory.

15. The use of compounds according to any of Claims 1 to 10 for producing a medicament for the treatment and/or prophylaxis of impairments of perception, concentration, learning and/or memory.

16. Medicament according to Claim 13 for the treatment and/or prophylaxis of impairments of perception, concentration, learning and/or memory.

## Revendications

1. Composés de formule : 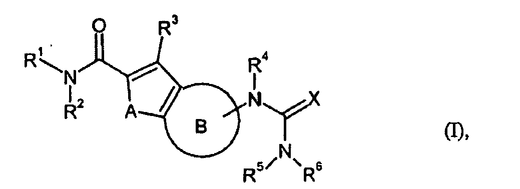 dans laquelle
R¹ est un groupe 1-aza-bicyclo[2.2.2]oct-3-yle,
R² représente l'hydrogène ou un reste alkyle en C₁ à C₆,
R³ représente l'hydrogène, un halogène, un groupe amino, hydroxy ou un reste alkyle en C₁ à C₆,
R⁴ représente l'hydrogène, un reste alkyle en C₁ à C₆ qui est éventuellement substitué par un reste choisi entre hydroxy, halogéno, cyano, alkoxy en C₁ à C₆, trifluorométhyle, trifluorométhoxy,
R⁵ représente l'hydrogène ou un reste alkyle en C₁ à C₆, ou bien
R⁴ et R⁵ forment, conjointement avec les atomes d'azote auxquels ils sont liés, un hétérocycle pentagonal ou hexagonal qui est éventuellement substitué avec jusqu'à 2 substituants choisis, indépendamment l'un de l'autre, entre alkyle en C₁ à C₆, acyle en C₁ à C₄, oxo, thioxo,
R⁶ représente (i) l'hydrogène, (ii) un reste alkyle en C₁ à C₆, (iii) un reste cycloalkyle en C₃ à C₈, (iv) un reste aryle en C₆ à C₁₀, (v) un reste hétéroaryle pentagonal à décagonal, (vi) un reste (aryle en C₆ à C₁₀)carbonyle, le reste (ii) étant éventuellement substitué avec un radical phényle, (alkoxy en C₁ à C₆)carbonyle ou alkoxy en C₁ à C₆ et les restes (iv), (v) et (vi) étant éventuellement substitués avec jusqu'à 3 restes choisis, indépendamment les uns des autres, entre alkyle en C₁ à C₆, hydroxyalkyle en C₁ à C₆, hétérocyclyle trigonal à octogonal, aryle en C₆ à C₁₀, hétéroaryle pentagonal à décagonal, hydroxy, halogéno, cyano, alkoxy en C₁ à C₆, acyle en C₁ à C₆, trifluorométhyle, trifluorométhoxy, nitro, amino, alkylamino en C₁ à C₆, acylamino en C₁ à C₆, ou bien
R⁵ et R⁶ forment, conjointement avec l'atome d'azote auquel ils sont liés, un hétérocycle trigonal à décagonal qui est éventuellement substitué avec un radical alkyle en C₁ à C₆ ou hydroxyalkyle en C₁ à C₆,
A représente l'oxygène, l'azote ou le soufre,
X représente l'oxygène ou le soufre,
le noyau B est un noyau benzo ou un noyau pyrido dont chacun est éventuellement substitué par des restes de la série halogéno, cyano, trifluorométhyle, trifluorométhoxy, nitro, amino, alkyle en C₁ à C₆ et alkoxy en C₁ à C₆,
ainsi que les produits de solvatation, les sels ou les produits de solvatation des sels de ces composés.

2. Composés suivant la revendication 1, de formule (I) dans laquelle :
R¹ est un groupe 1-aza-bicyclo[2.2.2]oct-3-yle,
R² représente l'hydrogène ou un reste alkyle en C₁ à C₆,
R³ représente l'hydrogène, un halogène, un groupe amino, hydroxy ou un reste alkyle en C₁ à C₆,
R⁴ représente l'hydrogène, un reste alkyle en C₁ à C₆ qui est éventuellement substitué par un reste choisi entre hydroxy, halogéno, cyano, alkoxy en C₁ à C₆, trifluorométhyle, trifluorométhoxy,
R⁵ représente l'hydrogène ou un reste alkyle en C₁ à C₆, ou bien
R⁴ et R⁵ forment, conjointement avec les atomes d'azote auxquels ils sont liés, un hétérocycle pentagonal ou hexagonal qui est éventuellement substitué avec jusqu'à 2 substituants choisis, indépendamment l'un de l'autre, entre alkyle en C₁ à C₆, acyle en C₁ à C₄, oxo, thioxo,
R⁶ représente (i) l'hydrogène, (ii) un reste alkyle en C₁ à C₆, (iii) un reste cycloalkyle en C₃ à C₈, (iv) un reste aryle en C₆ à C₁₀, (v) un reste hétéroaryle pentagonal à décagonal, le reste (ii) étant éventuellement substitué avec un radical phényle ou alkoxy en C₁ à C₆ et les restes (iv) et (v) étant éventuellement substitués avec jusqu'à 3 restes choisis, indépendamment les uns des autres, entre alkyle en C₁ à C₆, hydroxyalkyle en C₁ à C₆, hétérocyclyle trigonal à octogonal, aryle en C₆ à C₁₀, hétéroaryle pentagonal à décagonal, hydroxy, halogéno, cyano, alkoxy en C₁ à C₆, acyle en C₁ à C₆, trifluorométhyle, trifluorométhoxy, nitro, amino, alkylamino en C₁ à C₆, acylamino en C₁ à C₆, ou bien
R⁵ et R⁶ forment, conjointement avec l'atome d'azote auquel ils sont liés, un hétérocycle trigonal à octogonal qui est éventuellement substitué avec un radical alkyle en C₁ à C₆ ou hydroxyalkyle en C₁ à C₆,
A représente l'oxygène, l'azote ou le soufre,
X représente l'oxygène ou le soufre, et
le noyau B est un noyau benzo ou un noyau pyrido dont chacun est éventuellement substitué par des restes de la série halogéno, cyano, trifluorométhyle, trifluorométhoxy, nitro, amino, alkyle en C₁ à C₆ et alkoxy en C₁ à C₆,
ainsi que les produits de solvatation, les sels ou les produits de solvatation des sels de ces composés.

3. Composés suivant l'une des revendications 1 et 2, de formule (I) dans laquelle :
R¹ est un groupe 1-aza-bicyclo[2.2.2]oct-3-yle,
R² représente l'hydrogène ou un reste alkyle en C₁ à C₄,
R³ représente l'hydrogène, un halogène, un groupe amino, hydroxy ou un reste alkyle en C₁ à C₄,
R⁴ représente l'hydrogène, un reste alkyle en C₁ à C₄ qui est éventuellement substitué par un reste choisi entre hydroxy, halogéno, cyano, alkoxy en C₁ à C₃, trifluorométhyle, trifluorométhoxy,
R⁵ représente l'hydrogène ou un reste alkyle en C₁ à C₄, ou bien
R⁴ et R⁵ forment, conjointement avec les atomes d'azote auxquels ils sont liés, un hétérocycle pentagonal ou hexagonal qui porte éventuellement jusqu'à 2 substituants choisis, indépendamment l'un de l'autre, entre alkyle en C₁ à C₆, acyle en C₁ à C₄, oxo, thioxo,
R⁶ représente (i) l'hydrogène, (ii) un reste alkyle en C₁ à C₄, (iii) un reste cycloalkyle en C₅ ou C₆, (iv) un reste phényle, (v) un reste hétéroaryle pentagonal ou hexagonal, (vi) un reste (aryle en C₆ à C₁₀) carbonyle, le reste (ii) étant éventuellement substitué avec un radical phényle, (alkoxy en C₁ à C₄) carbonyle ou alkoxy en C₁ à C₃ et les restes (iv), (v) et (vi) étant éventuellement substitués avec jusqu'à 3 restes choisis, indépendamment les uns des autres, entre alkyle en C₁ à C₄, hydroxyalkyle en C₁ à C₄, hétérocyclyle trigonal à octogonal, aryle en C₆ à C₁₀, hétéroaryle pentagonal à décagonal, hydroxy, fluoro, chloro, cyano, alkoxy en C₁ à C₃, acyle en C₁ à C₃, trifluorométhyle, trifluorométhoxy, nitro, amino, alkylamino en C₁ à C₃, acylamino en C₁ à C₃, ou bien
R⁵ et R⁶ forment, conjointement avec l'atome d'azote auquel ils sont liés, un hétérocycle trigonal à décagonal qui est éventuellement substitué avec un radical alkyle en C₁ à C₃ ou hydroxyalkyle en C₁ à C₃,
A représente l'oxygène ou le soufre,
X représente l'oxygène,
le noyau B est un noyau benzo ou un noyau pyrido dont chacun est éventuellement substitué par des restes de la série chloro, fluoro, cyano, trifluorométhyle, trifluorométhoxy, amino, alkyle en C₁ à C₄ et alkoxy en C₁ à C₄,
ainsi que les produits de solvatation, les sels ou les produits de solvatation des sels de ces composés.

4. Composés suivant l'une des revendications 1 à 3, de formule (I) dans laquelle :
R¹ est un groupe 1-aza-bicyclo[2.2.2]oct-3-yle,
R² représente l'hydrogène ou un reste alkyle en C₁ à C₄,
R³ représente l'hydrogène, un halogène, un groupe amino, hydroxy ou un reste alkyle en C₁ à C₄,
R⁴ représente l'hydrogène ou un reste alkyle en C₁ à C₄ qui est éventuellement substitué par un reste choisi entre hydroxy, alkoxy en C₁ à C₃, trifluorométhyle, trifluorométhoxy,
R⁵ représente l'hydrogène ou un reste alkyle en C₁ à C₄, ou bien
R⁴ et R⁵ forment, conjointement avec les atomes d'azote auxquels ils sont liés, un hétérocycle pentagonal ou hexagonal qui est éventuellement substitué avec jusqu'à 2 substituants choisis, indépendamment l'un de l'autre, entre alkyle en C₁ à C₆, acyle en C₁ à C₄, oxo, thioxo,
R⁶ représente (i) l'hydrogène, (ii) un reste alkyle en C₁ à C₄, (iii) un reste cycloalkyle en C₅ ou C₆, (iv) un reste phényle, (v) un reste hétéroaryle pentagonal ou hexagonal, le reste (ii) étant éventuellement substitué avec un radical phényle et les restes (iv) et (v) étant éventuellement substitués avec jusqu'à 3 restes choisis, indépendamment les uns des autres, entre alkyle en C₁ à C₄, hydroxyalkyle en C₁ à C₄, hydroxy, chloro, fluoro, cyano, alkoxy en C₁ à C₃, acyle en C₁ à C₆, trifluorométhyle, trifluorométhoxy, amino, alkylamino en C₁ à C₃, acylamino en C₁ à C₃, ou bien
R⁵ et R⁶ forment, conjointement avec l'atome d'azote auquel ils sont liés, un hétérocycle pentagonal ou hexagonal qui est éventuellement substitué avec un radical alkyle en C₁ à C₃ ou hydroxyalkyle en C₁ à C₃,
A représente l'oxygène, l'azote ou le soufre,
X représente l'oxygène, et
le noyau B est un noyau benzo ou un noyau pyrido dont chacun est éventuellement substitué par des restes de la série chloro, fluoro, cyano, trifluorométhyle, trifluorométhoxy, amino, alkyle en C₁ à C₄ et alkoxy en C₁ à C₄,
ainsi que les produits de solvatation, les sels ou les produits de solvatation des sels de ces composés.

5. Composés suivant l'une des revendications 1 à 4, de formule (I) dans laquelle :
R¹ est un groupe 1-aza-bicyclo[2.2.2]oct-3-yle,
R² à R⁴ représentent l'hydrogène,
R⁵ représente l'hydrogène ou un reste alkyle en C₁ à C₄, ou bien
R⁴ et R⁵ forment, conjointement avec les atomes d'azote auxquels ils sont liés, un hétérocycle pentagonal ou hexagonal qui est éventuellement substitué avec jusqu'à 2 substituants choisis, indépendamment l'un de l'autre, entre alkyle en C₁ à C₄, acyle en C₁ à C₄, oxo, thioxo,
R⁶ représente (i) l'hydrogène, (ii) un reste alkyle en C₁ à C₄, (iii) un reste cycloalkyle en C₅ ou C₆, (iv) un reste phényle, (v) un reste pyridyle, (vi) un reste (aryle en C₆ à C₁₀)carbonyle, le reste (ii) étant éventuellement substitué avec un radical phényle, (alkoxy en C₁ à C₄)carbonyle ou alkoxy en C₁ à C₃ et les restes (iv), (v) et (vi) étant éventuellement substitués avec jusqu'à 3 restes choisis, indépendamment les uns des autres, entre alkyle en C₁ à C₄, hydroxyalkyle en C₁ à C₄, hétérocyclyle trigonal à octogonal, aryle en C₆ à C₁₀, hétéroaryle pentagonal à décagonal, hydroxy, fluoro, chloro, cyano, alkoxy en C₁ à C₃, acyle en C₁ à C₃, trifluorométhyle, trifluorométhoxy, nitro, amino, alkylamino en C₁ à C₃, acylamino en C₁ à C₃, ou bien
R⁵ et R⁶ forment, conjointement avec l'atome d'azote auquel ils sont liés, un hétérocycle trigonal à décagonal qui est éventuellement substitué avec un radical alkyle en C₁ à C₃ ou hydroxyalkyle en C₁ à C₃,
A représente l'oxygène ou le soufre,
X représente l'oxygène,
le noyau B est un noyau benzo,
ainsi que les produits de solvatation, les sels ou les produits de solvatation des sels de ces composés.

6. Composés suivant l'une des revendications 1 à 5, de formule (I) dans laquelle :
R¹ est un groupe 1-aza-bicyclo[2.2.2]oct-3-yle,
R² représente l'hydrogène,
R³ représente l'hydrogène, le chlore, le fluor, un groupe amino ou un reste alkyle en C₁ à C₃,
R⁴ représente l'hydrogène, un reste méthyle ou éthyle, les restes méthyle et éthyle étant éventuellement substitués par un reste choisi entre hydroxy, méthoxy, éthoxy, trifluorométhyle, trifluorométhoxy, ou bien
R⁴ et R⁵ forment, conjointement avec les atomes d'azote auxquels ils sont liés, un hétérocycle pentagonal ou hexagonal qui est éventuellement substitué avec jusqu'à 2 substituants choisis, indépendamment l'un de l'autre, entre alkyle en C₁ à C₃, acyle en C₁ à C₄, oxo, thioxo,
R⁵ représente l'hydrogène ou un reste alkyle en C₁ à C₃,
R⁶ représente (i) l'hydrogène, (ii) un reste alkyle en C₁ à C₄, (iii) un reste cyclopentyle, cyclohexyle, (iv) un reste phényle, (v) un reste benzyle, (vi) un reste phénéthyle, les restes (iv) à (vi) étant éventuellement substitués avec jusqu'à 3 restes choisis, indépendamment les uns des autres, entre hydroxy, chloro, fluoro, cyano, méthoxy, éthoxy, acyle en C₁ à C₄, trifluorométhyle, trifluorométhoxy, amino, alkylamino en C₁ à C₃,
A représente l'oxygène ou le soufre,
X représente le soufre, et
le noyau B est un noyau benzo qui est éventuellement substitué par des restes de la série chloro, fluoro, cyano, trifluorométhyle, trifluorométhoxy, alkyle en C₁ à C₄ , méthoxy et éthoxy,
ainsi que les produits de solvatation, les sels ou les produits de solvatation des sels de ces composés.

7. Composés de formule : 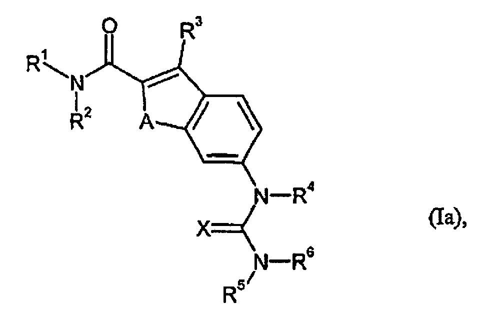 dans laquelle :
R¹ à R⁶, A et X ont les définitions indiquées dans les revendications 1 à 6, ainsi que les produits de solvatation, les sels ou les produits de solvatation des sels de ces composés.

8. Composés de formule : 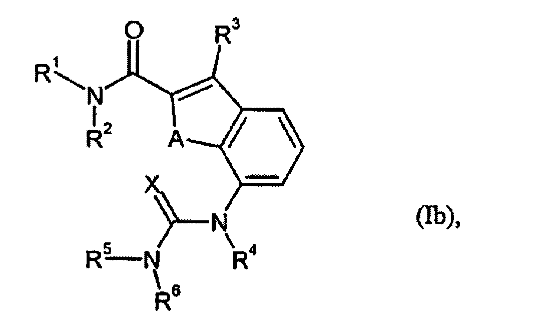 dans laquelle :
R¹ à R⁶, A et X ont les définitions indiquées dans les revendications 1 à 6, ainsi que les produits de solvatation, les sels ou les produits de solvatation des sels de ces composés.

9. Composés de formule : 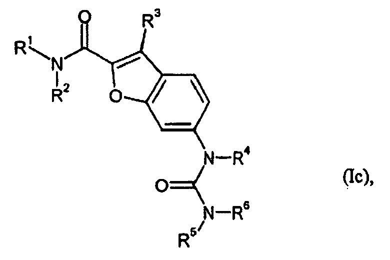 dans laquelle :
R¹ à R⁶ ont les définitions indiquées dans les revendications 1 à 6, ainsi que les produits de solvatation, les sels ou les produits de solvatation des sels de ces composés.

10. Composés de formule : 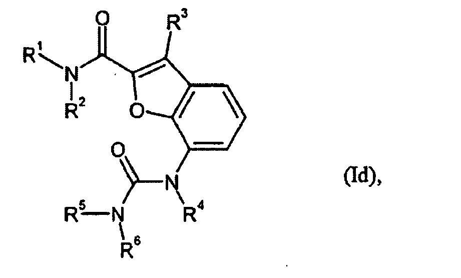 dans laquelle:
R¹ à R⁶ ont les définitions indiquées dans les revendications 1 à 6, ainsi que les produits de solvatation, les sels ou les produits de solvatation des sels de ces composés.

11. Procédé de production de composés suivant les revendications 1 à 10, dans lequel on fait réagir des composés de formule : 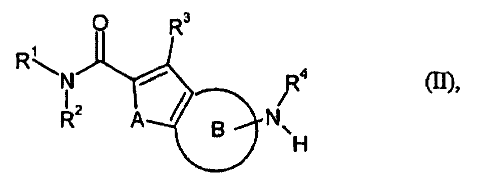 dans laquelle :
R¹ à R⁴, A et B ont les définitions indiquées dans les revendications 1 à 10,
avec des composés de formule : 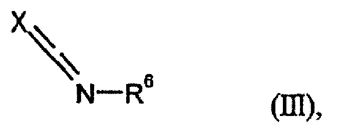
dans laquelle :
X et R⁶ ont les définitions indiquées dans les revendications 1 à 5,
et on transforme éventuellement les composés (I) résultants avec (a) les solvants correspondants et/ou (b) des bases ou des acides en leurs produits de solvatation, leurs sels ou les produits de solvatation des sels.

12. Composés suivant l'une des revendications 1 à 10, destinés au traitement et/ou à la prophylaxie de maladies.

13. Médicament contenant au moins un composé suivant l'une des revendications 1 à 10 et au moins un support ou excipient principalement non toxique, pharmaceutiquement acceptable.

14. Utilisation de composés suivant l'une des revendications 1 à 10, pour la préparation d'un médicament destiné à améliorer la perception, la capacité de concentration, la capacité d'apprentissage et/ou la capacité de mémoire.

15. Utilisation de composés suivant l'une des revendications 1 à 10, pour la préparation d'un médicament destiné au traitement et/ou à la prophylaxie de troubles de la perception, de la capacité de concentration, de la capacité d'apprentissage et/ou de la capacité de mémoire.

16. Médicament suivant la revendication 13, destiné au traitement et/ou à la prophylaxie de troubles de la perception, de la capacité de concentration, de la capacité d'apprentissage et/ou de la capacité de mémoire.
